# EUROPÄISCHE PATENTSCHRIFT

(11) **EP 4 149 395 B1**
(45) Veröffentlichungstag und Bekanntmachung des Hinweises auf die Patenterteilung: **16.08.2023**
(21) Anmeldenummer: 22757496.9
(22) Anmeldetag: 20.07.2022
(51) Int. Cl.: A61F 2/38

(54) **MEDIAL STABILISIERENDE KNIEENDOPROTHESE**
MEDIALLY STABILIZING KNEE PROSTHESIS
PROTHÈSE DE GENOU STABILISATEUR MÉDIAL

(30) Priorität: 28.07.2021 DE 102021119663
(43) Veröffentlichungstag der Anmeldung: 22.03.2023
(73) Patentinhaber: Aesculap AG, 78532 Tuttlingen/Donau (DE)
(72) Erfinder: RICHTER, Berna, 78570 Mühlheim (DE); ALTERMANN, Brigitte, 78532 Tuttlingen (DE); GRUPP, Thomas, 78588 Denkingen (DE); LANG, Rachel, 52000 Chaumont (FR); VOUAUX, Alexis, 52800 Poulangy (FR); BLANC, Steeven, 52000 Chaumont (FR)
(74) Vertreter: Winter, Brandl - Partnerschaft mbB
(86) Internationale Anmeldenummer: PCT/EP2022/070351
(87) Internationale Veröffentlichungsnummer: WO 2023/006545

(56) Entgegenhaltungen:
- EP-B1- 2 323 594
- DE-T2- 69 629 531
- US-A- 5 964 808

## Beschreibung

### Technisches Gebiet

Die vorliegende Offenbarung betrifft eine medial stabilisierende Knieendoprothese. Desweiteren wird ein zugehöriges Inlay vorgeschlagen. Die vorliegende Offenbarung liegt auf dem technischen Gebiet der Gesamtknie-Arthroplastie (engl. "total knee arthroplasty"; oder kurz: "TKA") bzw. der Knietotalendoprothese (kurz: Knie-TEP), wobei es sich um den vollständigen Ersatz eines verschlissenen oder verletzten Kniegelenks handelt. Das Einsatzgebiet der vorliegend offenbarten medial stabilisierenden Knieendoprothese beinhaltet sowohl die Indikation eines vorhanderen hinteren (bzw. posterioren) Kreuzbandes (lat./engl. "posterior cruciate ligament"; oder kurz: "PCL") als auch die Indikation eines abwesenden hinteren Kreuzbandes.

Der vorliegenden Offenbarung liegt als eine wesentliche Zielsetzung zugrunde, eine Knieendoprothese bereitzustellen, welche einem Patienten weitgehend die ihm gewohnten, natürlichen Bewegungsabläufe wie mit dem zuvor noch gesunden Knie ermöglichen. Zur Anatomie des Knies ist bekannt, dass ein Femur bzw. Oberschenkelknochen als ein oberer Beinabschnitt mit einer Tibia bzw. einem Schienbein als einem unteren Beinabschnitt im Knie durch ein, in stark vereinfachender Betrachtung, taschenmesserartiges Scharniergelenk verbunden ist. Dabei bezeichnet der Begriff der Flexion bzw. der Kniebeugung allgemein den (Knie-)Flexions-Winkel zwischen einem Femur und einer Tibia, wenn in einer Sagittalebene bzw. in einem Sagittalschnitt (durch ein jeweiliges Bein) betrachtet.

Heutzutage werden die Patienten in der Gesamtknie-Arthroplastie immer jünger und möchten damit weiterhin sportlich aktiv sein können. Jedoch werden im Stand der Technik seitens der Patienten als wiederkehrend auftretende Probleme mit bekannten Knieendoprothesen häufig deren Instabilität, eine mangelnde Mobilität sowie Schmerzen im anterioren (bzw. vorderen) Bereich des Knies beklagt. Die zugrundeliegenden Gründe können vielfachen Ursprungs sein: Zunächst ist eine mangelnde Stabilität, insbesondere in einem Bereich mittlerer Flexion, insbesondere in einem Flexions-Winkel (-Bogen) zwischen 20 Grad und 60 Grad (Flexion), zu nennen. Weiterhin können mangelnde Bewegungsmöglichkeiten der Knieendoprothese bei einem Bereich tiefer Flexion zugrundliegen. Ferner kann eine mangelnde Fähigkeit zu einer auf der medialen versus der lateralen Seite der Knieendoprothese idealerweise unterschiedlichen Mobilität ursächlich sein.

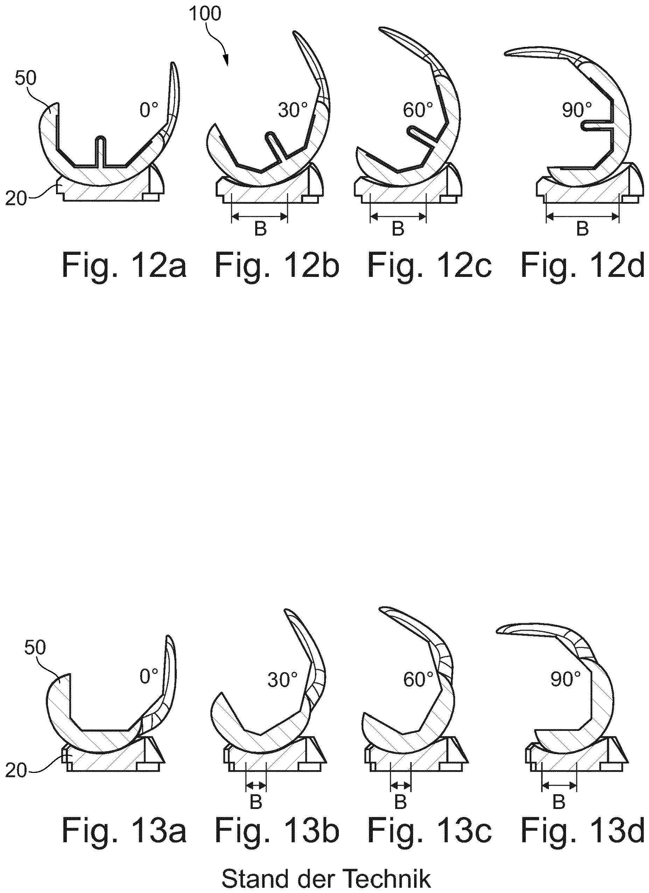

Die natürlichen Bewegungsabläufe im gesunden Knie bzw. dessen natürliche Kinematik sind bspw. mittels einer Magnetresonanzbildgebungsmethode durch die Wissenschaftler M.A.R. Freeman und V. Pinskerova genau untersucht. Hierzu wird auf deren wissenschaftliche Publikation "The movement of the knee studied by magnetic resonance imaging" [Clin. Orthop. Relat. Res. 2003;(410) 35-43] verwiesen, die hiermit durch Verweis ausdrücklich zum Bestandteil der vorliegenden Offenlegungsschrift gemacht wird. Insbesondere findet sich darin eine schematische Darstellung der medialen und lateralen Kontaktstelle auf der Tibia bei Flexion im natürlichen Kniegelenk (siehe Figur 12). Demzufolge finden natürliche Knieaktivitäten hauptsächlich bei einem gebeugten Flexions-Winkel zwischen 10 Grad und 120 Grad (Flexion), weiter bis ca. 135 Grad (Flexion) und in Ausnahmen extreme Flexion bis zu 165 Grad Flexions-Winkel statt. Die natürlichen Gelenkflächen (bzw. Artikulationsflächen) der (paarigen) Femur-Kondylen (bzw. Gelenkfortsätzen oder Gelenkknorren) des Femurs sind über diesen Flexions-Winkel (-Bogen) im Sagittalschnitt kreisförmig; dabei drehen sie sich um ihre Mitte.

Die natürliche mediale (bzw. zur Körpermitte hin angeordnete, zur Mitte hin gelegene) Femur-Kondyle bewegt sich nicht in einer antero-posterioreller Richtung (bzw. vordere-hintere Richtung). Demzufolge tritt beim natürlichen Knie ein Versatz nach posterior, orthopädisch als sogenannter "Roll-Back" bezeichnet, medial nicht auf. Die natürliche laterale (bzw. seitliche, von der Körpermitte abgewandte) Femur-Kondyle neigt zum "Roll-back", was in der natürlichen Kinematik eine innere tibiale Rotation mit Flexion auslöst. In einer natürlichen Situation mit einem nicht-gebeugten Bein, entsprechend einem im Wesentlichen gestreckten Flexions-Winkel (-Bogen) zwischen 0 Grad (d.h. voller Ausdehnung) bis hin zu 10 Grad bis 30 Grad, wird die tibiale Rotation mit Flexion gekoppelt. Die natürlichen Gelenkflächen sind medial mit einer anterioren (bzw. vorderen) Femur-Facette bzw. Femur-Schräge, welche einen größeren Radius aufweist, ausgebildet. Damit bildet diese mit einer nach oben geneigten Tibia-Facette bzw. Tibia-Facette ein Gelenk aus. Lateral rollt die Femur-Kondyle nach vorne auf das Vorderhorn (anteriore Horn des Außenmeniskus). Eine patientenabhängige Flexion über 120 Grad hinaus kann aktiv und/oder passiv erreicht werden. Medial rollt das Femur auf das Hinterhorn (posteriore Horn des Außenmeniskus). Lateral rutschen das Femur und das Hinterhom über die posteriore Tibia. Im gesunden Kniegelenk kommt es bei einem gebeugten Flexions-Winkel (-Bogen) zwischen 30 Grad und 90 Grad (Flexion) zu einer posterioren Translation des Innen- und Außenmeniskus und der lateralen Femur-Kondyle. Dabei bleibt die mediale Femur-Kondyle weitgehend stationär oder translatiert geringfügig nach anterior.

Zusammenfassend bedeutet diese vorstehend im Detail beschriebene Kinematik des natürlichen Kniegelenks, dass sich während der Kniebewegung das Femur auf der Tibia viel weniger medial als lateral bewegt. Dieser Umstand wird fachsprachlich auch als "medialer Drehpunkt" (engl. "medial pivot") bezeichnet. Die Drehachse des Femurs liegt auf der medialen Seite der Tibia.

Auf Basis dieser Erkenntnis wurden zuletzt Knieendoprothesen mit einem weitestgehend festgelegten medialen Drehpunkt des Femurs entwickelt, welche als "Medial-Pivot Design" (medialer-Drehpunkt-Konstruktion) bekannt sind. Diese Art der Bewegung wird im Stand der Technik durch die Kombination einer asymmetrisch ausgebildeten Femur-Lagerfläche eines Femur-Kondylen-Paars zusammen mit einer asymmetrisch ausgebildeten Tibia-Lagerfläche zur gemeinsamen Ausbildung des Gelenks derartiger Knieendoprothesen erreicht. Dabei ist die asymmetrisch ausgebildete Tibia-Lagerfläche zumeist in Form eines sogenannten "Inlays" (bzw. Einsatzes, Meniskus-Teils) fest an einem distalen Tibia-Teil angeordnet bzw. fest mit diesem verbunden, um als Oberfläche bzw. Gleitfläche für die Femur-Lagerfläche zu wirken. Damit bildet sich eine gesamthaft asymmetrisch geformte Tibiakomponente aus.

Beispielsweise offenbart die EP 2 323 594 B1 ein solches Prothesenkniegelenk, welches zur Nachahmung der natürlichen Kinematik des Knies ein einteiliges Tibia-Teil mit einer asymmetrisch ausgebildeten konkaven Tibia-Lagerfläche und ein einteiliges Femur-Teil mit einer gleichfalls asymmetrisch ausgebildeten kondylenförmigen Femur-Lagerfläche umfasst. Dabei ist das darin offenbarte Prothesenkniegelenk gemäß dem "Medial-Pivot Design" (medialer-Drehpunkt-Konstruktion), zusammenfassend, derart gestaltet, dass Verlagerungen der asymmetrischen Femur-Lagerfläche nach posteriorer Richtung in Reaktion auf eine zunehmende Beugung des Knies zu einer Rotation des Femur-Teils in Bezug auf das Tibia-Teil um eine externe Rotationsachse und um Flexions-Winkel innerhalb eines bestimmten Bereichs führen. Gleichzeitig bleibt ein Kontakt der asymmetrischen Tibia-Lagerfläche und der asymmetrischen Femur-Lagerfläche aufrechterhalten.

DE 696 29 531 T2 offenbart eine Kniegelenkprothese mit einer femoralen Komponente, einem tibialen Plateau und einem tibialen Lagerelement. Das Design und die Geometrie der Artikulationsflächen der Kondylenelemente der femoralen Komponente und des tibialen Lagerelements sind derart, dass die Kontaktfläche zwischen den Artikulationsflächen während der Flexion der Prothese maximiert wird. Dies wird erreicht, indem der koronale Radius der Lagerflächen der femoralen Komponente in der Richtung von anterior nach posterior entlang der Lagerfläche vergrößert wird. d.h. der koronale Radius der Lagerflächen der femoralen Komponente vergrößert wird, wenn die femorale Komponente von Extension zu Flexion übergeht.

US 5 964 808 A betrifft eine Knieprothese. Dabei kann wenigstens die Stirnfläche des medialen Kondylenabschnitts einer femoralen Komponente kongruent mit der Stirnfläche einer medialen Kavität in der oberen Fläche einer tibialen Komponente sein, so dass ein vollständiger Oberfläche-zu-Oberfläche-Kontakt zwischen der Stirnflächeder medialen Kavität in der oberen Oberfläche der tibialen Komponente und der Stirnfläche des medialen kondylären Abschnitts der femoralen Komponente über einen beträchtlichen Teil des Flexionsbereichs des Kniegelenks vorgesehen ist.

Jedoch bestehen bei den Lösungen aus dem Stand der Technik einige Nachteile:
Zunächst ist der feste mediale Drehpunkt nicht für alle Aktivitäten physiologisch. Ferner bleibt unberücksichtigt, dass das Knie mehr Stabilität im gestreckten Zustand (d.h. Flexion von 0 bis wenigen Grad) denn im gebeugten Zustand benötigt. Ein weiterer Nachteil besteht darin, dass diese Ausführungen auf der medialen Seite eine Kugelpfanne bzw. ein Kugelgelenk aufweisen, während auf der lateralen Seite eine eher flächig ausgeprägte Abstützung erfolgt. Das bedeutet, dass die Femur-Lagerfläche mit der Tibia-Lagerfläche, insbesondere dem Inlay, (quasi) vollständig kongruent bzw. kurvenkonform ist. Demzufolge dreht sich das Femur nur auf der medialen Seite, was zumindest den überwiegenden Teil bzw. Abschnitt, ggf. den vollständigen Teil, der Flexion bzw. des Flexions-Winkel(-Bogen)s betrifft. Somit bleibt der Drehpunkt bzw. das Drehzentrum stabil auf derselben Stelle für alle Aktivitäten bzw. Bewegungsabläufe. Demzufolge ist das "Medial-Pivot Design" (die medialer-Drehpunkt-Konstruktion) für den überwiegenden Anteil der, zumal jungen, Patienten als zu einschränkend für den Lebensalltag zu bewerten. Neben dem funktionellen Thema besteht mittelfristig das Risiko der Degradation der Gleitfläche durch Überlastung.

Eine Reihe noch weiterer Nachteile ergibt sich aus dem Umstand, dass die asymmetrisch ausgebildete Femur-Lagerfläche und die asymmetrisch ausgebildete Tibia-Lagerfläche als zwei Komponenten einer Kombination zusammenwirken und somit gut aufeinander abgestimmt bzw. passend zueinander ausgeformt, insbesondere dimensioniert, sein müssen.

Zunächst ergibt sich bei der medialer-Drehpunkt-Konstruktion eine Problematik hinsichtlich einer Kompatibilität zwischen verschiedenen Größen einerseits der Tibia des Patienten bzw. zumindest einer zugehörig passenden Tibia-Lagerfläche, insbesondere des Inlays, und andererseits des Femurs des Patienten bzw. zumindest einer zugehörig passenden Femur-Lagerfläche. Dabei ist es für den Chirurgen wichtig, in der Lage zu sein, jeder individuellen Situation des bzw. Indikation für den Patienten optimal entsprechen zu können. Insofern kommt es häufiger vor, dass bei einem individuellen Patienten das Femur und die Tibia im Vergleich unterschiedliche Größen aufweisen, beispielsweise können eine große Femurgröße (Skala 7) auf eine kleine Tibia (Skala 5) treffen. Jedoch bedingt die medialer-Drehpunkt-Konstruktion medial eine hohe Kongruenz bzw. ein hohes Maß an Formpassung zwischen einerseits der Tibia-Lagerfläche, insbesondere dem Inlay, und andererseits der Femur-Lagerfläche. Demzufolge ist auf der medialen Seite die Kompatibilität der Knieendoprothese nach der medialer-Drehpunkt-Konstruktion erheblich eingeschränkt, was die vorstehend beschriebene Situation des Patienten mit verschiedenen Größen einerseits der Tibia und andererseits des Femur anbelangt.

Weiter nachteilig kann der Chirurg nicht ein Standard-Femur-Teil bzw. eine Standard-Femur-Lagerfläche, vorzugsweise Kreuzband-erhaltend, zusammen mit einem medialer-Drehpunkt-Tibia-Teil bzw. einer medialer-Drehpunkt-Tibia-Lagerfläche, insbesondere einem medialer-Drehpunkt-Inlay, verwenden; oder, umgekehrt, nicht ein medialer-Drehpunkt-Femur-Teil bzw. eine medialer-Drehpunkt-Femur-Lagerfläche zusammen mit einem Standard-Tibia-Teil bzw. mit einer Standard-Tibia-Lagerfläche, insbesondere einem Standard-Inlay, vorzugsweise Kreuzband-erhaltend, verwenden. Dies hat nachteilhaft zur Folge, dass wenn der Chirurg fallweise, also in Abhängigkeit von der individuellen Situation des bzw. Indikation für den Patienten, beide Konzepte verwenden können möchte, er beide jeweilige Konstruktionen bzw. Kombinationen bzw. Paarungen passend für den Patienten vorhalten muss, also das Femur-Teil bzw. die Femur-Komponente zusammen mit dem Tibia-Teil bzw. der Tibia-Komponente einerseits gemäß der medialer-Drehpunkt-Konstruktion sowie andererseits gemäß der Standard-Konstruktion. Dies führt zu einem viel größeren Platzbedarf für vorzuhaltenden Bedarf und zu einer Erhöhung der Lagerkosten für den behandelnden Chirurgen bzw. das Krankenhaus.

Ähnlich gelagert kann sich ein noch weiterer Nachteil der Knieendoprothese nach der medialer-Drehpunkt-Konstruktion während der Operation bzw. intra-OP ergeben. Und zwar tritt mitunter der Fall ein, dass sobald die Knieendoprothese implantiert ist, sich in der realen Situation aufgrund des mechanischen Einflusses von Ligamenten, Sehnen, Weichgeweben, nicht das erforderliche bzw. gewünschte Soll-Maß an Stabilität erreicht wird, sondern sich entweder eine zu große Stabilität oder eine Instabilität feststellen lässt. Deshalb muss der Chirurg die Möglichkeit am eröffneten Knie haben, im Nachhinein zur Erreichung des Soll-Maßes an Stabilität entweder den Femur-Teil oder den Tibia-Teil zu verändern. Beispielsweise im Falle einer sich als zu groß herausstellenden Stabilität, wird der Chirurg die Maßnahme ergreifen wollen, die anfänglich implantierte medialer-Drehpunkt-Tibia-Lagerfläche, insbesondere das medialer-Drehpunkt-Inlay, auf die Standard-Tibia-Lagerfläche, insbesondere das Standard-Inlay, zu ändern bzw. für eine solche auszutauschen. Jedoch würde dies bedingen, dass in diesem Fall die asymmetrisch ausgebildete medialer-Drehpunkt-Femur-Lagerfläche nicht mehr kompatibel wäre, so dass der Chirurg zusätzlich auch den medialer-Drehpunkt-Femur-Teil auf einen Standard-Femur-Teil bzw. eine Standard-Femur-Lagerfläche ändern müsste. Damit ist der operative Eingriff für den Chirurgen wie auch für den Patienten in negativer Weise verlängert.

Diese Aufgaben werden in einem ersten Aspekt offenbarungsgemäß durch die Merkmale des auf eine medial stabilisierende Knieendoprothese gerichteten Anspruchs 1 gelöst. Dadurch werden die vorstehend beschriebenen Nachteile überwunden.

Eine medial stabilisierende Knieendoprothese für eine Gesamtknie-Arthroplastie unter Erhalt oder unter Dissektion des hinteren Kreuzbandes hat (bzw. weist auf): eine Femur-Lagerfläche, welche an einem zur Festlegung an einem distalen Ende eines Femurs eingerichteten Femur-Teil zur Ausrichtung nach distal vorgesehen ist; und eine Tibia-Lagerfläche, welche an einem zur Festlegung an einem proximalen Ende einer Tibia eingerichteten Tibia-Teil zur Ausrichtung nach proximal vorgesehen ist. Insbesondere kann dabei die Tibia-Lagerfläche als eine proximale Fläche eines an dem Tibia-Teil proximal angeordneten und/oder anordbaren Inlays ausgebildet sein. Die Femur-Lagerfläche weist dabei eine konvexe mediale Femur-Kondylen-Lagerfläche und eine konvexe laterale Femur-Kondylen-Lagerfläche auf. Die Tibia-Lagerfläche weist dabei eine konkave mediale Lagerschale und eine konkave laterale Lagerschale auf und ist zur Aufnahme und verschiebbaren Gleitlagerung der Femur-Lagerfläche ohne einen festen medialen Drehpunkt der Femur-Lagerfläche in der medialen Lagerschale eingerichtet. Dabei bilden offenbarungsgemäß die mediale Lagerschale und die laterale Lagerschale zueinander eine asymmetrische Tibia-Lagerfläche aus. Kumulativ bilden die mediale Femur-Kondylen-Lagerfläche und die laterale Femur-Kondylen-Lagerfläche zueinander eine nicht-asymmetrische oder quasisymmetrische Femur-Lagerfläche aus. Dabei stimmen die mediale Femur-Kondylen-Lagerfläche und die laterale Femur-Kondylen-Lagerfläche zumindest in einem anterioren Femur-Kondylen-Radius jeweiliger anteriorer Flächenabschnitte überein. Dabei bildet die asymmetrische Tibia-Lagerfläche eine außen umlaufende proximale Höhenkontur aus. Dabei weist die Höhenkontur einen anterioren Höhenkonturabschnitt mit zumindest einem anterioren Höhenpunkt und einen posterioren Höhenkonturabschnitt mit zumindest einem posterioren Höhenpunkt auf. Dabei ist der anteriore Höhenpunkt gegenüber dem posterioren Höhenpunkt hinsichtlich zumindest einer Sagittalebene proximal erhöht. Dabei bildet ein antero-medialer Höhenkonturabschnitt des anterioren Höhenkonturabschnitts an der medialen Lagerschale einen proximal am meisten erhöhten anterioren Höhenpunkt als einen antero-medialen Gipfelpunkt aus.

Der Begriff "nicht-asymmetrisch" bezeichnet offenbarungsgemäß eine ohne Asymmetrie ausgebildete (Femur-) Lagerfläche. Insbesondere ist damit eine quasisymmetrische, weiter bevorzugt symmetrische, (Femur-) Lagerfläche gemeint. Dabei versteht der Fachmann, dass beim Vergleich der medialen Femur-Kondylen-Lagerfläche und der lateralen Femur-Kondylen-Lagerfläche auf die mittigen konvexen Kontaktflächen der verschiebbaren Gleitlagerung abzustellen ist. Sprich, die spezifische Ausgestaltung von peripheren bzw. außermittigen bzw. dezentralen Randbereichen (an) der Femur-Lagerfläche bzw. um die Femur-Lagerfläche herum spielt bei der Bewertung eines Grades an Symmetrie gar keine oder höchstens eine untergeordnete Rolle. Auch kommt es vorliegend bei der Beurteilung nicht auf etwaige fertigungsbedingte Toleranzabweichungen an.

Allgemein versteht der Fachmann, dass Asymmetrie beim Vergleich zwischen einer medialen Lagerfläche bzw. Lagerschale und einer lateralen Lagerfläche bzw. Lagerschale sich auf unterschiedliche Ausgestaltungen mit erheblichen Abweichungen hinsichtlich der jeweiligen (Krümmungs-) Radien und/oder der Führungslinien hinsichtlich eines kinematischen Bewegungsablaufes bezieht.

Mit anderen Worten, soll der Begriff "symmetrisch" nicht im absoluten oder spitzfindigen Sinne, sondern mit technischem Augenmaß bzw. mit Blick auf die Funktion der Gleitlagerung und die Auswirkung insb. auf die Kinematik verstanden sein. Demzufolge ist auf die Gesamtanmutung der Femur-Kondylen-Lagerflächen im Vergleich der lateralen zu der medialen Seite abzustellen. Von dem Begriff "nichtasymmetrisch" sind auch bevorzugte Ausführungsformen mit geringfügigen Abweichungen der (Krümmungs-) Radien und/oder der Führungslinien umfasst. Im Vergleich der medialen Femur-Kondylen-Lagerfläche und der lateralen Femur-Kondylen-Lagerfläche soll von dem Begriff "nicht-asymmetrische Femur-Lagerfläche" eine solche bevorzugte Ausführungsform miteingeschlossen sein, bei welcher lediglich geringfügige Abweichungen der (Krümmungs-) Radien der Nachahmung bzw. Berücksichtigung der natürlichen bzw. anatomischen Unterschiede bei beiden Femur-Kondylen (Innenseite versus Außenseite) beim Patienten dienen.

Insbesondere soll das Merkmal "nicht-asymmetrische Femur-Lagerfläche" jedoch eine vorbekannte Ausgestaltung einer medial (hoch)stabilen Knieendoprothese mit einem festgelegten medialen Drehpunkt des Femurs, welche als "Medial-Pivot Design" (medialer-Drehpunkt-Konstruktion) bekannt ist, ausschließen. Wie einleitend beschrieben, betrifft letztere Ausgestaltung die Kombination einer asymmetrisch ausgebildeten Femur-Lagerfläche eines Femur-Kondylen-Paars mit einer asymmetrischen Tibia-Lagerfläche. Insbesondere fällt in Abgrenzung zum Stand der Technik eine derartige vorbekannte Ausgestaltung mit einer asymmetrisch ausgebildeten Femur-Lagerfläche zur Ausbildung eines medialen Kugelgelenks als Gleitlagerung der (sphärischen) medialen Femur-Kondylen-Lagerfläche auf einer (sphärischen) medialen Lagerschale einer Tibia-Lagerfläche nicht unter das vorbezeichnete Merkmal der "nicht-asymmetrischen Femur-Lagerfläche".

Die asymmetrische Ausgestaltung der Tibia-Lagerfläche der offenbarungsgemäßen Knieendoprothese bewirkt zunächst den Vorteil einer wünschenswerten medialen Stabilisierung auf der medialen Seite aufgrund einer, im Vergleich zu einer symmetrischen Tibia-seitigen Gleitfläche, höheren, damit verbesserten, Kongruenz bzw. Formpassung der Femur-Lagerfläche zur Tibia-Lagerfläche. Insbesondere erlaubt die offenbarungsgemäße Knieendoprothese ein Optimum einer medial erhöhten Kongruenz, welche die Stabilität des Bewegungsablaufes im künstlichen Kniegelenk verbessert, bei gleichzeitig lateral hohen kinematischen Freiheitsgraden, was zusammengenommen aktivere und sogar sportliche Bewegungsabläufe unterstützt. Zusammenfassend ergibt sich daraus für viele Indikationen bzw. eine für eine große Patientengruppe eine nach allen Kriterien weitestgehend optimale Gesamtkinematik und verbesserte Beweglichkeit.

Die offenbarungsgemäße Lösung und die sich daraus ableitenden technischen Vorteile ergeben sich aus dem Zusammenwirken der Tibia-Lagerfläche und der Femur-Lagerfläche in der besonderen Kombination von deren jeweiligen spezifischen Formgestaltungen zur Ausbildung der verschiebbaren Gleitlagerung. Der der vorliegenden Offenbarung zugrundeliegende technische Lösungsgedanke fusst in der Kombination einer nicht-asymmetrischen, vorzugsweise einer im Wesentlichen symmetrischen, Femur Lagerfläche mit einer asymmetrischen Tibia-Lagerfläche, eingerichtet, um die verschiebbare Gleitlagerung, im Sinne der Bewegungsabläufe eines (künstlichen) Kniegelenkes, auszubilden.

Somit ist es nicht weiter erheblich, in welcher konkreten Weise die Tibia-Lagerfläche an dem Tibia-Teil zur Ausrichtung nach proximal vorgesehen bzw. mit dem Tibia-Teil verbunden bzw. in dem Tibia-Teil (integral) ausgebildet ist. Demzufolge können im Sinne der vorliegenden Offenbarung Gesichtspunkte hinsichtlich der Anordnung der Tibia-Lagerfläche an dem Tibia-Teil weniger betrachtet bleiben. Vorzugsweise kann die Knieendoprothese in der Bauweise mit einer festen Auflage der Tibia-Lagerfläche ausgeführt sein. Alternativ vorzugsweise kann die Knieendoprothese in der Bauweise mit einer beweglichen Auflage der Tibia-Lagerfläche ausgeführt sein.

So kann einerseits eine Ausgestaltung bevorzugt sein, bei welcher die Tibia-Lagerfläche als eine proximale (Ober-) Fläche des Tibia-Teils bzw. in dem Tibia-Teil ausgebildet ist.

Andererseits kann gerade eine solche Ausgestaltung bevorzugt sein, bei welcher die Tibia-Lagerfläche als eine proximale (Ober-) Fläche in einem separat ausgeformten Inlay (bzw. einem meniskusähnlichen Knieprothesenteil, vorzugsweise aus einem thermoplastischen Kunststoff bzw. einem Duroplasten, wie bspw. Polyethylen, ausgebildet) ausgebildet ist. Sprich, bei dieser bevorzugten Ausführungsform auf Basis eines separaten Inlays bietet dieses eine Gleitfläche aus Polyethylen zwischen dem Femur-Teil und dem Tibia-Teil (bzw. zwischen der Ober- und der Unterschenkel-Komponente) der Knieendoprothese. Diese bevorzugte Ausführungsform bietet den Vorteil, dass das als Gleitfläche für das künstliche Kniegelenk dienende Inlay den natürlichen Gelenkspalt mit den Menisken ersetzt.

Es sind Varianten der Ausgestaltung hinsichtlich einer Anordnung des Inlays an dem Tibia-Teil gleichermaßen denkbar, vorzugsweise dass das Inlay auf dem Tibia-Teil schwimmend gelagert ist, beweglich verbunden bzw. verwendbar oder in fester Weise verbunden bzw. verwendbar, beispielsweise aufgeschraubt, pressgepasst, und dergleichen.

Insbesondere ergibt sich ein erstgenannter Vorteil der vorliegenden Offenbarung unter Gesichtspunkten der Kinematik der offenbarungsgemäßen Knieendoprothese. So gelingt es mittels der offenbarungsgemäßen Knieendoprothese, die einleitend beschriebene natürliche Kinematik des gesunden Knies weitestgehend nachzuempfinden. Dabei zeichnet sich die natürliche Kinematik insbesondere durch eine zwischen der medialen Seite und der lateralen Seite des Knies asymmetrische Bewegung aus. Offenbarungsgemäß wird im Falle eines ausgestreckten bzw. nur leicht gebeugten Knies, entsprechend einem im Wesentlichen gestreckten Flexions-Winkel (-Bogen) zwischen 0 Grad (d.h. bei voller Ausdehnung) bis hin wenigen Winkelgraden, die erforderliche hohe Stabilität bewirkt, und zwar unabhängig von der medialen und lateralen Seite. Darüberhinaus behält die offenbarungsgemäße Knieendoprothese in einem Bereich mittlerer Flexion, insbesondere in einem Flexions-Winkel (-Bogen) zwischen 20 Grad und 40 Grad (Flexion), eine immer noch hohe mediale Stabilität bei. Dabei weist sie auch eine vorteilhafte antero-laterale Stabilität auf. Gleichzeitig erlaubt die offenbarungsgemäße Knieendoprothese in vorteilhafter Weise, dass die postero-laterale Stabilität ein wenig abnehmen kann, so dass der lateralen Femur-Kondylen-Lagerfläche ein sogenannter "Roll-back", d.h. ein Versatz nach posterior bzw. ein Zurückrollen, bei gleichzeitiger Möglichkeit zur Rotation ermöglicht wird. Dies erlaubt erheblich verbesserte Freiheitsgrade bei dynamischen Bewegungsabläufen bei gleichzeitig aufrechterhaltener Stabilität im künstlichen Kniegelenk. Schliesslich bei hoher Flexion gelingt es offenbarungsgemäß, dass zum Nachempfinden der natürlichen Kinematik die postero-mediale Stabilität nur wenig abnimmt und gleichzeitig sich die postero-laterale Stabilität weitgehend reduziert. Demzufolge gelingt es, dass der "Rollback" auf der medialen Seite gering ausfällt, während er aus der lateralen Seite demgegenüber hoch ausfallen kann, wobei gleichzeitig eine signifikante Drehung des Femurs bzw. Oberschenkelknochens ermöglicht ist.

Als zweitgenannter Vorteil hinsichtlich der gewünschten Nachahmung der natürlichen Kinematik ist zu nennen, dass die asymmetrische Ausgestaltung der Tibia-Lagerfläche der offenbarungsgemäßen Knieendoprothese eine vorteilhafte Dissoziation der kinematischen Freiheitsgrade auf der medialen versus der lateralen Seite ermöglicht.

Ein drittgenannter Vorteil betrifft den Punkt einer mittels der offenbarungsgemäßen Knieendoprothese reduzierten Komplexität für den Operateur sowie die Lagervorhaltung in der Klinik. Aufgrunddessen, dass die mediale Femur-Kondylen-Lagerfläche und die laterale Femur-Kondylen-Lagerfläche zueinander eine nicht-asymmetrische, im Wesentlichen symmetrische, insbesondere eine hinsichtlich einer dazwischen angeordneten Sagittalebene spiegelsymmetrische, Femur-Lagerfläche ausbilden, kann das Femur-Teil unabhängig von der betroffenen Knieseite, also sowohl für das linke Knie als auch für das rechte Knie, verwendet werden. Damit halbiert sich die Anzahl an klinikseitig vorzuhaltenden bzw. in der Operation zu differenzierenden Prothesenteilen, wodurch sich die Komplexität und die Kosten der Lagervorhaltung bzzw. Operationsplanung vorteilhaft verringern. Ohnehin müssen in einer Knieprothesen-Produktfamilie herstellerseitig wie klinikseitig mehrere Größen bzw. Dicken für Femur-Teile sowie für Tibia-Teile bzw. Inlays verfügbar vorgehalten werden, um den vielfältigen Patientensituationen operativ entsprechen zu können. Somit stellt die offenbarungsgemäße Knieendoprothese für den Operateur eine die Komplexität absenkende und kostengünstigere Lösung dar.

Als viertgenannter Vorteil ist zu erwähnen, dass die Offenbarung eine vorteilhafte Kompatibilität hinsichtlich der Knieendoprothesenfamilie über den integrationsseitig erforderlichen Größenbereich leistet. Aufgrund der Variabilität der Anatomie müssen unterschiedliche Größen von Tibia-Teil (bzw. Tibia-Lagerflächen) mit unterschiedlichen Femur-Teilen (bzw. Femur-Lagerflächen) arbeiten. Die erforderliche Kompatibilität über den indikationsseitig erforderlichen Größenbereich stellt eine enorme technische Herausforderung dar. Wie einleitend beschrieben, ist die erforderliche Kompatibilität insbesondere im Falle der vorbekannten Ausgestaltungen mit einem einzigen Radius zur Ausbildung eines (medialen) Kugelgelenks problematisch. Insofern ist die Problematik bei diesem Stand der Technik mit einem einzigen Radius sogar verstärkt, insofern aufgrund der konstruktiv hohen Kongruenz zwischen der Femur-Lagerfläche zu der Tibia-Lagerfläche auf der medialen Seite die Kompatibilität der Größen ganz erheblich eingeschränkt ist. Hier schafft die offenbarungsgemäße Knieendoprothese, insbesondere vorstehend beschriebene bevorzugte Ausführungsform, eine technisch vorteilhafte Lösung. Insofern liegt keine solche Beschränkung in Folge der zueinander symmetrischen Femur-Kondylen-Lagerflächen, die jeweilig mit mehreren Radien pro Größenindikation ausgebildet sein können. Auf diese Weise wird offenbarungsgemäß ein erhöhter Knochenverlust vermieden, da die Verankerungskonstruktionen mit kleineren Radien auf kleinere Größenindikationen passend ausgeführt werden können.

Als fünftgenannter Vorteil ist zu erwähnen, dass die vorliegende Offenbarung eine Implantation der offenbarungsgemäßen Knieendoprothese bzw. eine Gesamtknie-Arthroplastie sowohl unter Erhalt als auch unter Dissektion des hinteren Kreuzbandes ermöglicht. Somit ist dem Operateur ein breites Indikationsfeld und ein flexibles Eingehen auf unterschiedliche Indikationen ermöglicht. Eine erste Indikation betrifft eine Konstellation beim Patienten, bei welcher das Innen- und Außenband, ggf. auch das hintere Kreuzband ausreichend stabil sind, so dass die idealerweise zu erhaltenden Bänder nach erfolgreicher Implantation der offenbarungsgemäßen Knieendoprothese weiterhin die Bewegungen des Knies, d.h. Beugen (Flexion), Strecken (Extension) und Drehen (Rotation), koordinieren können. Eine weitere Indikation betrifft eine Konstellation beim Patienten, bei welcher der Operateur das, gegebenenfalls von Arthrose vorgeschädigte, hinteren Kreuzband während der Operation vor dem Einbau der offenbarungsgemäßen Knieendoprothese entfernt.

Darüberhinaus ergeben sich noch zwei weitergenannte offenbarungsgemäße Vorteile, wie im Folgenden beschrieben:
Offenbarungsgemäß definiert die asymmetrische Tibia-Lagerfläche der Knieendoprothese eine außen umlaufende proximale Höhenkontur bzw. bildet eine solche aus. Dabei hat die Höhenkontur einen anterioren Höhenkonturabschnitt, welcher zumindest einen anterioren Höhenpunkt aufweist bzw. auf welchem eine Vielzahl anteriorer Höhenpunkte liegen. Die Höhenkontur hat ferner einen posterioren Höhenkonturabschnitt, welche zumindest einen posterioren Höhenpunkt aufweist bzw. auf welchem eine Vielzahl posteriorer Höhenpunkte liegen. Dabei ist der anteriore Höhenpunkt gegenüber dem posterioren Höhenpunkt proximal erhöht, hinsichtlich zumindest einer Sagittalebene bzw. wenn auf eine Sagittalebene projiziert. Bevorzugt kann der anteriore Höhenkonturabschnitt gegenüber dem posterioren Höhenkonturabschnitt, insbesondere vollständig, proximal erhöht sein. Dadurch, dass der gesamte anteriore Höhenkonturabschnitt gegenüber dem gesamten posterioren Höhenkonturabschnitt nach proximal (bzw. beim aufrechtstehenden Patienten: nach oben) erhöht ist, ist der anteriore Höhenkonturabschnitt aus einer Rückansicht des Tibia-Teils, insbesondere des Inlays, frei sichtbar. Dies dient insbesondere einer vorteilhaft weiter verbesserten anterioren Stabilisierung.

Weiter offenbarungsgemäß bildet ein antero-medialer Höhenkonturabschnitt des anterioren Höhenkonturabschnitts an der medialen Lagerschale einen proximal am meisten erhöhten anterioren Höhenpunkt als einen antero-medialen Gipfelpunkt aus. Mit anderen Worten, ist dabei die Tibia-Lagerfläche seitens ihres (nach proximal ausgerichteten) Höhenprofils derart gestaltet, dass an der anterioren Seite ein (Hüll-)Flächenabschnitt der medialen Lagerschale besonders hochgezogen (bzw. nach proximal erhöht) ist, so dass zumindest dieser in einem (proximal) höchsten anterioren Höhenpunkt als einen antero-medialen Gipfelpunkt gipfelt bzw. zu einem solchen ansteigt. Demzufolge wird die mediale Femur-Kondylen-Lagerfläche besonders an der anterioren Seite der Knieendoprothese (gleit-)lagemd von der zugehörigen medialen Lagerschale der Tibia-Lagerfläche eingefasst, somit im Bewegungsablauf geführt bzw. stabilisiert. Die vorstehend hinsichtlich der Kinematik genannten Vorteile werden im Ergebnis verstärkt.

Gesamthaft bietet die offenbarungsgemäße Kombination einer zur medialen Stabilisierung asymmetrisch ausgebildeten Tibia-Lagerfläche eines Tibia-Teils, insbesondere eines Inlays, zusammen mit einer (quasi-) symmetrischen Femur-Lagerfläche mit (im Wesentlichen) beidseitig gleich ausgebildeten Femur-Kondylen-Lagerflächen eine ausgewogene Balance bei der Erreichung diverser einzelner, teils gegenläufiger, technischer Zielen. Insbesondere wird einerseits dem essentiellen technischen Ziel eines naturgetreuen kinematischen Verhaltens bzw. Bewegungsablaufes, selbst für Patienten mit hoher kinematischer Anforderung bzgl. Stabilität und "Range of Motion" (d.h. Bewegungsspielraum bei z.B. Sport, asiatischer Lebensstil), nach erfolgter Implantation der offenbarungsgemäßen Knieendoprothese bzw. einer Gesamtknie-Arthroplastie gedient. Und andererseits wird offenbarungsgemäß auch das weitere technische Ziel einer erhöhten Effizienz und verringerten Komplexität für den Operateur bzw. den Klinikablauf erheblich unterstützt.

Insofern die vorliegende Offenbarung eine technische Lösung ohne einen festen medialen Drehpunkt der Femur-Lagerfläche in der medialen Lagerschale betrifft, überwindet sie die einleitend zum Stand der Technik im Bereich des "Mediat-Pivot Designs" (der medialer-Drehpunkt-Konstruktion) beschriebenen grundsätzlichen Nachteile.

Vorliegend werden die Begriffe, welche der anatomischen Lage- und Richtungsbezeichnung dienen, fachbegrifflich bzw. anatomisch verwendet. So werden die Lage- und Richtungsbezeichnungen proximal [lat. proximus "nächster"; zum Körperzentrum (des Patienten, nicht des Chirurgen) hin (angeordnet bzw. ausgerichtet)]; im Gegensatz zu distal [lat. distare "entfernt sein"; vom Körperzentrum (des Patienten) entfernt] verwendet.

Ferner bedeutet, vorliegend auf ein Kniegelenk bzw. die Knieendoprothese bezogen, die Lage- und Richtungsbezeichnung medial [lat. medium "Mitte"; zur Medianebene hin] innen bzw. an der Innenseite des Knies (angeordnet bzw. zu dieser hin ausgerichtet). Im Gegensatz dazu bedeutet die Lage- und Richtungsbezeichnung lateral [lat. latus "Seite"; von der Medianebene weg, zur Seite hin] außen, an der Außenseite des Knies (angeordnet bzw. zu dieser hin ausgerichtet).

Die Lage- und Richtungsbezeichnung anterior [lat. ante "vor"] bedeutet vorderer, vorn liegend, was vorliegend ventral [zur Vorderseite, den Bauch betreffend] entspricht. Beim natürlichen Knie liegt somit die Knieschiebe [lat. Patella] anterior. Im Gegensatz dazu bedeutet die Lage- und Richtungsbezeichnung posterior [lat. post "hinter"] hinterer, hinten liegend, was vorliegend dorsal [am bzw. zum Rücken gelegen] entspricht.

Die drei zueinander orthogonalen anatomischen Raumachsen bzw. Richtungen werden wie vorliegend bezeichnet: Die Longitudinal-Achse verläuft in einer Höhenrichtung [Oberseite, oben ↔ Unterseite, unten], insb. beim aufrecht stehenden Patienten vertikal vom Kopfscheitel bis zur Fußsohle, hier vorzugsweise durch das Knie. Ferner die Sagittal-Achse [lat. sagitta "Pfeil"; anterior, vorne ↔ posterior, hinten] verläuft in einer Tiefenrichtung, insb. in Vorderansicht des aufrecht stehenden Patienten. Ferner noch die Transversal-Achse [medial, innen ↔ lateral, außen, bspw. rechts ↔ links) verläuft in einer Breitenrichtung, insb. in Vorderansicht des aufrecht stehenden Patienten.

Der anatomische Begriff einer Frontalebene bezeichnet eine zu einer Sagittalebene und einer Transversalebene jeweils senkrechte bzw. orthogonale Ebene. Dabei meint die Sagittalebene vorliegend insb. eine Ebene, welche den Femur bzw. die Tibia in eine rechte und eine linke (Knie-) Hälfte unterteilt. Die Transversalebene ist eine (bei einem aufrecht stehenden Patienten horizontale) Ebene, welche senkrecht zu einer Longitudinal-Achse, insbesondere einer solchen der Tibia, steht.

Eine kondylenförmige Fläche bezeichnet eine (Ober-)Fläche, welche an einem distalen Ende eines Femur-Teils angeordnet ist, welche die Form einer anatomischen bzw. natürlichen Femur-Kondyle nachempfindet.

Der Fachbegriff der (Kurven-) Konformität (bzw. konform) zwischen zwei Kurven bzw. offenbarungsgemäß zwischen (jeweiligen anliegenden Kurvenabschnitten) einer Tibia-Lagerfläche und einer Femur-Lagerfläche (wenn auf eine Schnittebene, insbesondere auf eine mediale und/oder zentrale und/oder laterale Sagittalebene, projiziert) meint, dass die zugehörigen Radien an einem Kontaktpunkt im Wesentlichen bzw. nominell einander entsprechen bzw. gleich sind. Insofern betrifft die Konformität ein Maß für die Lager-Formpassung zwischen (jeweiligen Kurvenabschnitten) der Tibia-Lagerfläche zu der Femur-Lagerfläche.

Hingegen führt ein Mangel an (Kurven-) Konformität zu einer Laxheit als Gegenbegriff, d.h. im Sinne von einem hohen Lagerspiel bzw. einer geringen Lager-Formpassung. Sprich, Laxheit bezeichnet ein Maß für eine Verlagerung bzw. einen Versatz und/oder für eine (Winkel-) Drehung und/oder für einen kinematischen Freiheitsgrad, welche(r) infolge eines Mangels an (Kurven-) Konformität zwischen (jeweiligen anliegenden Kurvenabschnitten) einer Tibia-Lagerfläche und einer Femur-Lagerfläche eintreten kann.

Der Fachbegriff der Außenrotation des Femurs bezeichnet die Rotation des Femurs um eine Außenrotationsachse, welche an der medialen Lagerschale des Tibia-Teils angeordnet ist und parallel zu der Längsachse des Tibia-Teils liegt.

Die Unteransprüche zeigen bevorzugte Weiterbildungen der Offenbarung.

Vorzugsweise, alternativ oder kumulativ, kann der anteriore Höhenpunkt, hinsichtlich zumindest einer Sagittalebene bzw. wenn auf eine Sagittalebene projiziert, eine anteriore Lagerhöhe ausbilden bzw. definieren. Dabei ist die anteriore Lagerhöhe auf einen proximal tiefsten Lager-Tiefpunkt in der (jeweils zugehörigen) konkaven medialen und/oder lateralen Lagerschale bezogen. Dabei bildet der posteriore Höhenpunkt eine posteriore Lagerhöhe aus bzw. definiert eine solche, hinsichtlich zumindest einer Sagittalebene bzw. wenn auf eine Sagittalebene projiziert. Dabei ist die posteriore Lagerhöhe, gleichfalls wie bereits die anteriore Lagerhöhe, den proximal tiefsten Lager-Tiefpunkt in der (jeweils zugehörigen) konkaven medialen und/oder lateralen Lagerschale bezogen. Bei dieser bevorzugten Ausführungsform ist die anteriore Lagerhöhe größer als die posteriore Lagerhöhe ist. Noch weiter bevorzugt ist die maximale anteriore Lagerhöhe an dem antero-medialen Gipfelpunkt ausgebildet. Auch bei dieser bevorzugten Ausführungsform werden die vorstehend hinsichtlich der Kinematik genannten Vorteile weiter verstärkt. Insbesondere wird die antero-mediale Stabilisierung noch weiter optimiert.

Vorzugsweise, alternativ oder kumulativ, kann die mediale Lagerschale der Knieendoprothese so ausgeformt sein, dass, wenn in einer medialen Sagittalebene betrachtet, ein Steigungsdreieck von dem anterioren Höhenpunkt mit der anterioren Lagerhöhe zu dem proximal tiefsten Lager-Tiefpunkt in der medialen Lagerschale aufgespannt ist. Dabei bildet das Steigungsdreieck einen an dem proximal tiefsten Lager-Tiefpunkt angeordneten medialen Steigungswinkel aus. Insbesondere bemisst der mediale Steigungswinkel 12 bis 32 Grad, weiter bevorzugt 17 bis 27 Grad, insbesondere ca. 22 Grad. Dieser bevorzugte Wertebereich bzw. Wert des medialen Steigungswinkels bietet ein Optimum hinsichtlich der antero-medialen Stabilisierung im künstlichen Kniegelenk.

Vorzugsweise, alternativ oder kumulativ, können, hinsichtlich der Femur-Lagerfläche der offenbarungsgemäßen Knieendoprothese, die mediale Femur-Kondylen-Lagerfläche und die laterale Femur-Kondylen-Lagerfläche, im Wesentlichen, kondylenförmig ausgebildet. Dadurch ist die Formgebung des Femur-Teils besonders optimiert.

Vorzugsweise, alternativ oder kumulativ, kann ein posteriorer (Kurven-) Abschnitt jeweils der medialen Femur-Kondylen-Lagerfläche und der lateralen Femur-Kondylen-Lagerfläche in einem Bereich von 0 bis 95 Grad Flexions-Winkel (-Bogen) mit einem gleichbleibenden bzw. konstanten posterioren Radius ausgebildet sein. Bei dieser bevorzugten Ausführungsform werden die vorstehend genannten Vorteile weiter vergrößert.

Vorzugsweise, alternativ oder kumulativ, kann ein distalster (bzw. am meisten distal angeordneter) Punkt der medialen Femur-Kondylen-Lagerfläche einen medialen Extensions-Lagerflächen-Kontaktpunkt, welcher mit der medialen Lagerschale der Tibia-Lagerfläche bei ausgestrecktem Bein bzw. ca. 0 Grad Flexions-Winkel (d.h. Extension) in Kontakt steht, ausbilden. Dabei kann insbesondere der mediale Extensions-Lagerflächen-Kontaktpunkt, wenn bei ausgestrecktem Bein bzw. ca. 0 Grad Flexions-Winkel (d.h. Extension) hinsichtlich einer Sagittalebene als Winkelsegment auf eine anteriore Kante der medialen Femur-Kondylen-Lagerfläche bezogen, bei 50 bis 70 Grad, weiter bevorzugt bei 58 bis 62 Grad, insbesondere bei ca. 60 Grad, angeordnet sein. Alternativ oder kumulativ kann insbesondere der mediale Extensions-Lagerflächen-Kontaktpunkt, wenn bei ca. 0 Grad Flexions-Winkel hinsichtlich einer Sagittalebene als Winkelsegment auf eine anteriore Kante der medialen Tibia-Lagerschale bezogen, bei 55 bis 75 Grad, weiter bevorzugt bei 63 bis 67 Grad, insbesondere bei ca. 65 Grad, angeordnet sein. Auch bei dieser bevorzugten Ausführungsform werden die vorstehend hinsichtlich der Kinematik genannten Vorteile weiter verstärkt. Insbesondere wird die mediale Stabilisierung noch weiter optimiert.

Noch weiter bevorzugt kann der mediale Extensions-Lagerflächen-Kontaktpunkt mit dem proximal tiefsten Lager-Tiefpunkt in der konkaven medialen und/oder lateralen Lagerschale zusammenfallen. Alle diese Merkmale fördern eine mediale Stabilisierung.

Insgesamt ergeben sich bei den vorstehenden bevorzugten Ausführungsformen der vorliegenden Offenbarung hinsichtlich der Betrachtung der Evolvente in vorteilhaft medial stabilisierender Weise ein stärker posterior angeordneter Extensions-Lagerflächen-Kontaktpunkt sowie hinsichtlich der medialen Lagerschale ein größerer anteriorer Radius und ein demgegenüber kleinerer posteriorer Radius.

Vorzugsweise, alternativ oder kumulativ, kann die konkave mediale Lagerschale, hinsichtlich einer medialen Sagittalebene, zumindest einen anterioren Flächenabschnitt mit einem ersten Radius, welcher einen antero-medialen Tibia-Lagerflächen-Radius ausbildet bzw. definiert, und zumindest einen posterioren Flächenabschnitt mit einem zum ersten Radius unterschiedlichen, vorzugsweise kleineren, zweiten Radius, welcher einen postero-medialen Tibia-Lagerflächen-Radius ausbildet bzw. definiert, ausbilden bzw. definieren. Dies dient einer weiterhin optimierten Kinematik und Beweglichkeit.

Insbesondere kann dabei der antero-mediale Tibia-Lagerflächen-Radius kongruent, insb. identisch, zu einem anterioren Femur-Kondylen-Radius ausgebildet sein. Dabei ist der anteriore Femur-Kondylen-Radius als ein dritter Radius eines anterioren Flächenabschnitts der medialen Femur-Kondylen-Lagerfläche ausgebildet bzw. definiert. Es kann weiter bevorzugt sein, dass der anteriore Femur-Kondylen-Radius bzw. dritte Radius gemäß einer Femur-Größenklasse für einen Patienten vorbestimmt ist. Dies dient einer weiterhin optimierten Kinematik, hinsichtlich Kongruenz und Beweglichkeit. Ferner dient dies einer Optimierung des klinischen Größenmanagements für eine Knieendoprothesenfamilie.

Alternativ oder kumulativ kann dabei insbesondere der postero-mediale Tibia-Lagerflächen-Radius kongruent, insb. identisch, zu einem posterioren Femur-Kondylen-Radius ausgebildet sein. Dabei ist der posteriore Femur-Kondylen-Radius als ein vierter Radius eines posterioren Flächenabschnitts der medialen Femur-Kondylen-Lagerfläche ausbildet bzw. definiert. Es kann weiter bevorzugt sein, dass der posteriore Femur-Kondylen-Radius bzw. der vierte Radius gemäß einer Femur-Größenklasse für einen Patienten vorbestimmt ist. Demzufolge werden Vorteile hinsichtlich einer positiven Kongruenz sowie einer auf der medialen Seite benötigten ausreichenden Stabilität erreicht. Ferner dient dies einem weiter optimierten klinischen Größenmanagement für eine Knieendoprothesenfamilie.

Vorzugsweise, alternativ oder kumulativ, kann die konkave laterale Lagerschale, hinsichtlich einer lateralen Sagittalebene, zumindest einen anterioren Flächenabschnitt mit einem fünften Radius, welcher einen antero-lateralen Tibia-Lagerflächen-Radius ausbildet bzw. definiert, und zumindest einen posterioren Flächenabschnitt mit einem zum fünften Radius unterschiedlichen, vorzugsweise größeren, sechsten Radius, welcher einen postero-lateralen Tibia-Lagerflächen-Radius ausbildet bzw. definiert, ausbilden bzw. definieren. Auch dies kann sich kinematisch positiv auswirken.

Insbesondere kann dabei der antero-laterale Tibia-Lagerflächen-Radius bzw. fünfte Radius kongruent, insb. identisch, zu dem (vorstehend definierten) anterioren Femur-Kondylen-Radius bzw. dem dritten Radius ausgebildet sein.

Alternativ oder kumulativ kann dabei insbesondere der postero-laterale Tibia-Lagerflächen-Radius bzw. sechste Radius größer als der (vorstehend definierte) posteriore Femur-Kondylen-Radius bzw. der vierte Radius ausgebildet sein. Dabei kann es weiter bevorzugt sein, dass der postero-laterale Tibia-Lagerflächen-Radius um zumindest 110 Prozent größer als der posteriore Femur-Kondylen-Radius bemessen ist. Insbesondere beträgt der postero-laterale Tibia-Lagerflächen-Radius einen größergleich 80 mm bemessenden Radius.

Aus der bevorzugten Anpassung des, insbesondere medialen, posterioren Tibia-Lagerflächen-Radius an die Größe des posterioren Femur-Kondylen-Radius folgert ein (nach proximal) stärker hochgezogener posteriorer Höhenkonturabschnitt (bzw. eine hohe posteriore Lippe). Dies dient zwar einer guten Stabilität, insbesondere für die mediale Seite. Jedoch benötigen die Patienten für einige (sportlichere) Aktivitäten mit tiefen Beugungen des Knies einen hohen Flexionsgrad. Damit ist erforderlich, dass femoraler Roll-back (etwas medial und insb. lateral) sowie Rotation stattfinden. Mit einer hohen posterioren Lippe können der Knochen und das Inlay bei hoher Flexion insb. auf der medialen Seite in Konflikt kommen, was zu Schmerzen und sehr eingeschränkten Bewegungen führen kann.

Zur Lösung des vorstehend beschriebenen Problems, kann, alternativ oder kumulativ, vorzugsweise an der medialen Lagerschale eine von dem (vorstehend definierten) posterioren Höhenpunkt schräg nach distal verlaufende postero-mediale Phase, vorgesehen sein. Dies dient dazu, um (von der äußeren Hüllfläche der medialen Lagerschale) peripheres Material wegzunehmen. Dabei steht die postero-mediale Phase, wenn in einer Sagittalebene projiziert, unter einem posterioren Phasenwinkel zu einer Transversalebene. Insbesondere kann die postero-mediale Phase mit einem posterioren Phasenwinkel von 30 bis 40 Grad, weiter bevorzugt von 33 bis 37 Grad, insbesondere von ca. 35 Grad, angeschrägt sein bzw. angefast sein. Damit wird in vorteilhafter Weise ein Konflikt zwischen dem Knochen und einem postero-medialen Höhenkonturabschnitt (bzw. einer proximalen Kante der posterioren Lippe der medialen Lagerschale) vermieden, welcher sonst bei hoher Flexion aufträte. Insbesondere unterstützt dieses Merkmal die Ansprüche von Patienten mit asiatischem Lebensstil bzw. Sportarten mit hoher Flexion. Demzufolge können unter Erhalt der guten Stabilität Knochenverschleiß oder Schmerzen an diesem postero-medialen Punkt im Vorfeld vermieden werden.

Vorzugsweise, alternativ oder kumulativ, kann an der lateralen Lagerschale eine an dem posterioren Höhenpunkt schräg nach distal verlaufende postero-laterale Phase vorgesehen sein. Vergleichbar der postero-medialen Phase dient dies dazu, um (von der äußeren Hüllfläche der lateralen Lagerschale) peripheres Material wegzunehmen. Dabei steht die postero-laterale Phase, wenn in einer Sagittalebene projiziert, unter einem posterioren Phasenwinkel zu einer Transversalebene. Insbesondere kann die postero-laterale Phase mit einem posterioren Phasenwinkel von 5 bis 15 Grad, weiter bevorzugt von 8 bis 12 Grad, insbesondere von ca. 10 Grad, ausgebildet sein. Alternativ oder kumulativ kann die postero-laterale Phase mit einem postero-lateralen Rundungsradius von 10 bis 14 mm, weiter bevorzugt von 11,2 bis 12,8 mm, insbesondere von ca. 12 mm, abgerundet sein. Auch diese Maßnahme beugt in vorteilhafter Weise Knochenverschleiß und Schmerzen vor.

Vorzugsweise, alternativ oder kumulativ, kann die Tibia-Lagerfläche eine, zentral zwischen der medialen Lagerschale und der lateralen Lagerschale vorgesehene, anteriore Patella-Ausbuchtung bzw. einen Patella(sehnen)-Ausschnitt mit einer (im Wesentlichen) konkaven äußeren Körperkontur ausbilden. Dabei kann insbesondere die Patella-Ausbuchtung kongruent zu einer für einen Patienten vorbestimmten Patella(sehnen)-Größenklasse ausgebildet sein. Alternativ oder kumulativ kann die Patella-Ausbuchtung einem statistisch-anthropometrisch vorbestimmten 50-Perzentil-Wert für eine Patella(sehnen)-Größenverteilung hinsichtlich einer repräsentativen, insb. Regionen-spezifischen, Bevölkerungspopulation entsprechen. Dies dient dazu, der Patella(sehne) einen verbesserten Bewegungsspielraum zur Ausübung ihrer anatomischen bzw. kinematischen Funktion zu bieten.

Insbesondere kann dabei die Patella-Ausbuchtung asymmetrisch ausgebildet sein. Dies dient dazu, die Ausbildung eines an der medialen Lagerschale proximal stärker als an der lateralen Lagerschale erhöhten Höhenkonturabschnittes zu ermöglichen. Insofern ist die Patella-Ausbuchtung asymmetrisch ausgebildet, um im zentralen Bereich der Tibia-Lagerschale einen zum antero-medialen Gipfelpunkt steileren Anstieg des anterioren Höhenkonturabschnittes auszubilden.

Vorzugsweise, alternativ oder kumulativ, können die mediale Femur-Kondylen-Lagerfläche und die laterale Femur-Kondylen-Lagerfläche zueinander eine hinsichtlich einer dazwischen angeordneten Sagittalebene, im Wesentlichen, spiegelsymmetrische Femur-Lagerfläche ausbilden. Dies dient allen fünf vorstehend ausführlich genannten Vorteilsgruppen.

Vorzugsweise, alternativ oder kumulativ, kann die mediale Femur-Kondylen-Lagerfläche zumindest in einem distal-medialen Flächenabschnitt einen medialen Femur-Kondylen-Lagerflächenradius, insbesondere zur Nachempfindung eines natürlichen medialen Femurkondylus-Radius, gleichbleibend (bzw. konstant) aufweisen. Dabei weist die laterale Femur-Kondylen-Lagerfläche zumindest in einem lateral-medialen Flächenabschnitt einen anderen lateralen Femur-Kondylen-Lagerflächenradius, insbesondere zur Nachempfindung eines natürlichen lateralen Femurkondylus-Radius, gleichbleibend (bzw. konstant) auf. Dabei können der mediale Femur-Kondylen-Lagerflächenradius und der laterale Femur-Kondylen-Lagerflächenradius voneinander geringfügig abweichen, nämlich maximal um einen Verhältnisfaktor von 0,8 bis 1,2, vorzugsweise von 0,9 bis 1,1, insbesondere von 0,95 bis 1,05. Damit ist eine noch weiter verbesserte Anpassung auf die anatomische Situation eines Patienten im Lichte der Offenbarung möglich.

Vorzugsweise, alternativ oder kumulativ, kann die Tibia-Lagerfläche zur Aufnahme und verschiebbaren Gleitlagerung der Femur-Lagerfläche entlang einer durch eine Vielzahl von Kontaktpunkten ausgebildeten Leitkrümmungslinie eingerichtet sein, so dass bei einer flektierenden Beugung der Knieendoprothese von 30 Grad auf ca. 90 ° Grad Flexions-Winkel ein spezifischer Bewegungsablauf [real (bspw. im Artikulator vor einer Operation und/oder nach Implantation im Patienten) und/oder virtuell (bspw. anhand der 3D-Finite-Elemente-Methode im Computermodell simulierbar)] ausgelöst wird bzw. evolviert bzw. bewirkt wird, wie anhand der folgenden (alternativen und insbesondere kumulativen) kinematischen (Unter-)Kriterien quantifizierbar:
(i) Dabei wird ferner vorzugsweise eine Außenrotation des Femur-Teils um eine zu einer Longitudinalachse des Tibia-Teils parallelen Außenrotationsachse, welche insbesondere an der medialen Lagerschale angeordnet ist, bewirkt
(ii) Dabei wird ferner vorzugsweise ein femoraler Roll-back bewirkt. Weiter vorzugsweise kann der femorale Roll-back kleinergleich minus 1,5 mm bei 30 Grad und/oder kleinergleich minus 6 mm bei 60 Grad und/oder kleinergleich minus 11 mm bei ca. 90 Grad Flexions-Winkel betragen [insofern Werte im negativen Bereich, wenn auf Roll-front-Achse, d.h. auf eine in Vorwärtsrichtung positive Koordinatenachse bezogen]. Entsprechend bedeutet dies, dass vorzugsweise ein Betragswert des femoralen Roll-backs vorzugsweise größergleich 1,5 mm bei 30 Grad und/oder größergleich 6 mm bei 60 Grad und/oder größergleich 11 mm bei ca. 90 Grad Flexions-Winkel betragen kann.
(iii) Weiter vorzugsweise kann die femorale Bewegung von einer (ohnehin minimalen) Roll-front in den (im Weiteren betragsmäßig zunehmenden) Roll-back in einem Umkehrbereich bei ca. 10 bis 20 Grad, insb. ca. 14 Grad, Flexions-Winkel wechseln.
(iv) Weiter vorzugsweise kann (bis zum Erreichen des Umkehrbereiches) bei ca. 10 bis 20 Grad, insb. ca. 14 Grad, Flexions-Winkel (d.h. in der geringen bzw. frühen Flexion) der femorale Roll-front kleinergleich 2 mm, insb. kleinergleich ca. 0,5 mm, betragen.

Alle vorstehend genannten alternativen oder kumulativen, unabhängigen, Merkmale können zur weiteren Optimierung der Kinematik konkret beitragen. Insb. kann, zumal je nach Einzelfall des Patienten bzw. einer Patientengruppe mit spezifischen Anamnesen, Diagnosen, Bedürfnissen etc., ein einzelnes (Unter-)Merkmal oder mehrere bzw. alle (Unter-)Merkmale in Kombination bzw. synergistisch bevorzugt oder auch gerade nicht bevorzugt sein.

Vorzugsweise, alternativ oder kumulativ, kann die Tibia-Lagerfläche zur Aufnahme und verschiebbaren Gleitlagerung der Femur-Lagerfläche eingerichtet sein, so dass bei einer flektierenden Beugung der Knieendoprothese von 30 Grad auf ca. 90 Grad Flexions-Winkel ein spezifischer Bewegungsablauf [real und/oder virtuell (bspw. im Computermodell simulierbar)] bewirkt wird, wie anhand der folgenden (alternativen und insbesondere kumulativen) kinematischen (Unter-)Kriterien quantifizierbar:
- eine mediale posteriore Bewegung um delta 1mm bis 4mm bewirkt wird;
   und/oder;
- eine lateral posteriore Bewegung um delta 8 mm bis 11 mm bewirkt wird.

Das bewirkt in vorteilhafter Weise eine stabile Standbeinphase und freie Schwungphase.

Als ein zweiter Aspekt der vorliegenden Offenbarung wird ein zugehöriges Inlay, welches für eine offenbarungsgemäße Knieendoprothese eingerichtet ist, vorgeschlagen. Dabei ist, wie bereits vorstehend ausgeführt, das Inlay an dem Tibia-Teil der Knieendoprothese proximal angeordnet, insbesondere integral mit dem Tibia-Teil ausgebildet, und/oder anordbar. Dabei bildet eine proximale Fläche des Inlays die eine konkave mediale Lagerschale und eine konkave laterale Lagerschale aufweisende Tibia-Lagerfläche aus. Die Tibia-Lagerfläche ist zur Aufnahme und verschiebbaren Gleitlagerung der eine konvexe mediale Femur-Kondylen-Lagerfläche und eine konvexe laterale Femur-Kondylen-Lagerfläche aufweisenden Femur-Lagerfläche ohne einen festen medialen Drehpunkt der Femur-Lagerfläche in der medialen Lagerschale eingerichtet. Dabei bilden die mediale Lagerschale und die laterale Lagerschale zueinander eine asymmetrische Tibia-Lagerfläche aus; und bilden die mediale Femur-Kondylen-Lagerfläche und die laterale Femur-Kondylen-Lagerfläche zueinander eine nicht -asymmetrische Femur-Lagerfläche aus. Insbesondere kann die nicht-asymmetrische Femur-Lagerfläche als eine hinsichtlich einer, zwischen der medialen Femur-Kondylen-Lagerfläche und der lateralen Femur-Kondylen-Lagerfläche angeordneten bzw. zentralen, Sagittalebene spiegelsymmetrische Femur-Lagerfläche ausgebildet sein. Dabei stimmen die mediale Femur-Kondylen-Lagerfläche und die laterale Femur-Kondylen-Lagerfläche zumindest in einem anterioren Femur-Kondylen-Radius jeweiliger anteriorer Flächenabschnitte überein. Dabei bildet die asymmetrische Tibia-Lagerfläche eine außen umlaufende proximale Höhenkontur aus. Dabei weist die Höhenkontur einen anterioren Höhenkonturabschnitt mit zumindest einem anterioren Höhenpunkt und einen posterioren Höhenkonturabschnitt mit zumindest einem posterioren Höhenpunkt auf. Dabei ist der anteriore Höhenpunkt gegenüber dem posterioren Höhenpunkt hinsichtlich zumindest einer Sagittalebene proximal erhöht. Dabei bildet ein antero-medialer Höhenkonturabschnitt des anterioren Höhenkonturabschnitts an der medialen Lagerschale einen proximal am meisten erhöhten anterioren Höhenpunkt als einen antero-medialen Gipfelpunkt aus.

Dies bietet insbesondere Vorteile hinsichtlich einer separaten Fertigung (bspw. nicht-metallverarbeitend, Spritzguss etc.) des für die offenbarungsgemäße Knieendoprothese eingerichteten Inlays als solchem und hinsichtlich seines logistischen Materialflusses im und ab Werk bzw. in den klinischen Abläufen.

Im Nachfolgenden werden vorteilhafte Ausführungsbeispiele der vorliegenden Offenbarung unter Bezugnahme auf die begleitenden Figuren näher erläutert.

### Kurzbeschreibung der Figuren

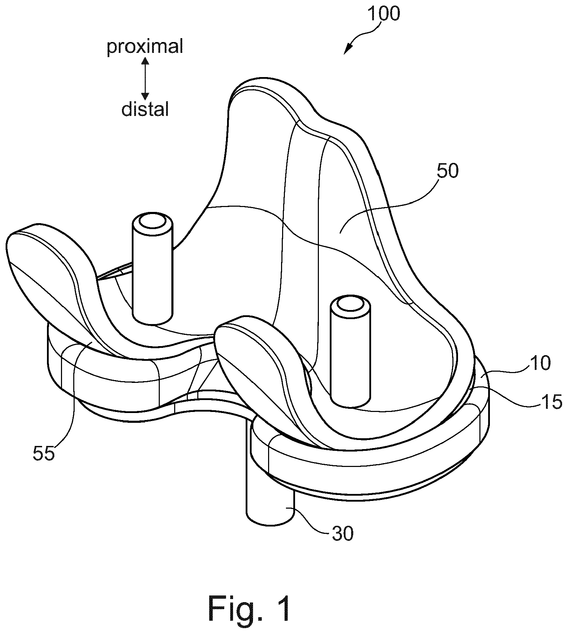

Fig. 1 ist eine perspektivische schematische Darstellung einer Knieendoprothese für eine Gesamtknie-Arthroplastie, gesehen von einem postero-lateralen Blickpunkt, insbesondere veranschaulichend eine gesamte Anordnung mit einem zur distalen Festlegung an einem Femur eingerichteten Femur-Teil mit einer Femur-Lagerfläche und einem, darunter bzw. dazu distal angeordneten, zur proximalen Festlegung an eine Tibia eingerichteten Tibia-Teil mit einer Tibia-Lagerfläche;

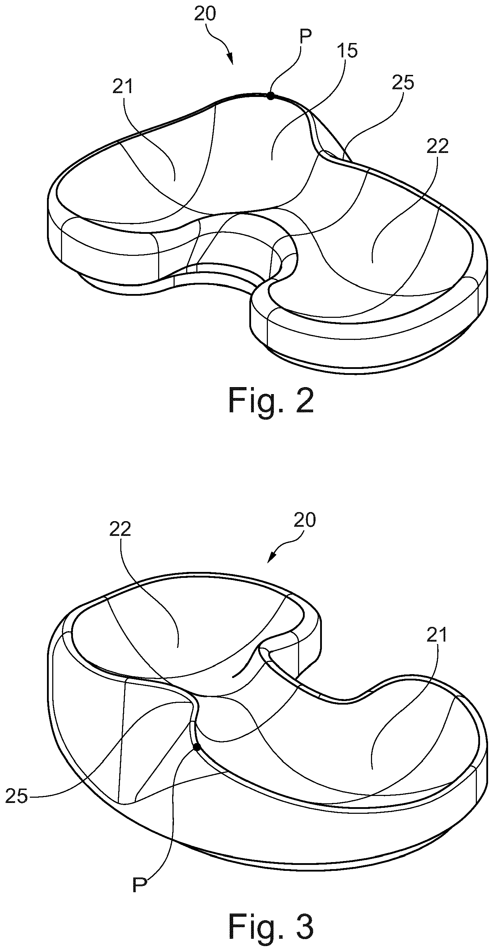

Fig. 2 ist eine perspektivische Aufsicht eines Inlays einer medial stabilisierenden Knieendoprothese gemäß einem bevorzugten Ausführungsbeispiel nach der vorliegenden Offenbarung, gesehen von einem postero-lateralen Blickpunkt, in einer Blickrichtung auf das Inlay als die nach proximal ausgerichtete Tibia-Lagerfläche (bzw. auf die Oberseite der Tibia bzw. des Tibia-Teils), insb. zur Veranschaulichung einer medialen Lagerschale und einer lateralen Lagerschale;
Fig. 3 ist eine (um 180° zu der Aufsicht der Fig. 2 im Raum gedrehte) perspektivische Aufsicht des Inlays der Knieendoprothose gemäß dem Ausführungsbeispiel, gesehen von einem antero-medialen Blickpunkt;

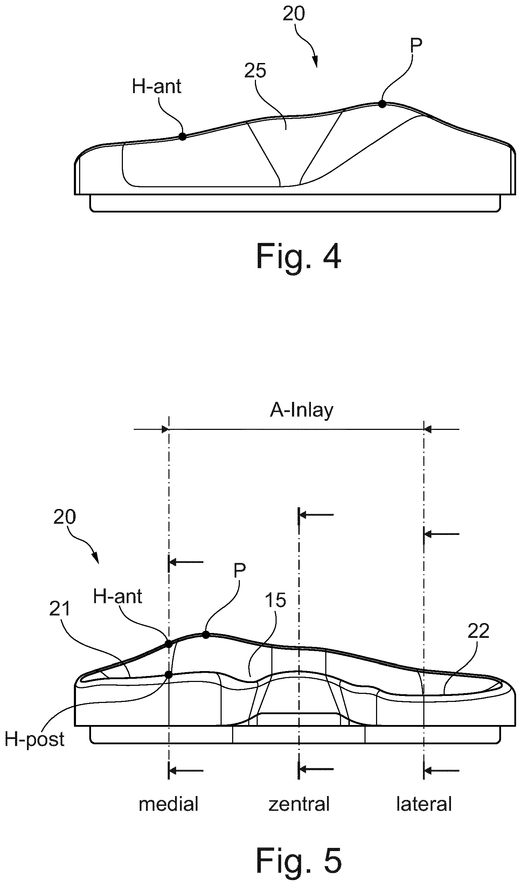

Fig. 4 ist eine anteriore Ansicht des Inlays der Knieendoprothose gemäß dem Ausführungsbeispiel;
Fig. 5 ist eine (um 180° zu der Ansicht der Fig. 4 im Raum gedrehte) posteriore Ansicht des Inlays der Knieendoprothose gemäß dem Ausführungsbeispiel, insb. zur Veranschaulichung der jeweiligen Lage eines medialen Querschnitts, eines zentralen Querschnitts sowie eines lateralen Querschnitts (insb. im Wesentlichen in einer Sagittalebene);

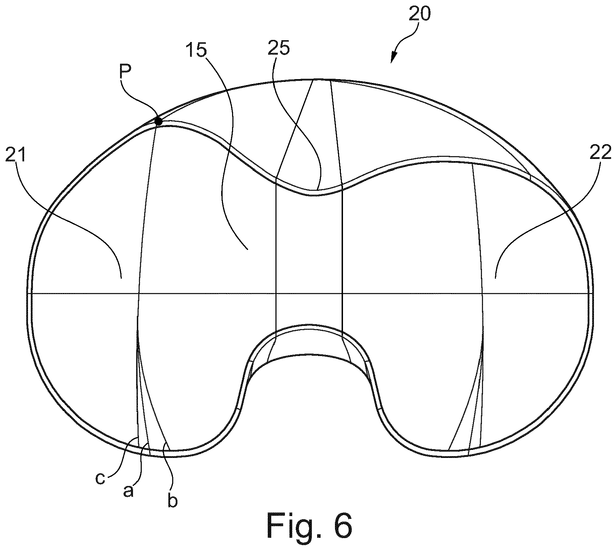

Fig. 6 ist eine Draufsicht des Inlays der Knieendoprothose gemäß dem Ausführungsbeispiel, gesehen von einem proximalen Blickpunkt, in einer Blickrichtung nach distal auf das Inlay (bzw. auf die Oberseite des Tibia-Teils), insb. zur Veranschaulichung von drei Varianten einer jeweiligen Leitkrümmungslinie, ausgebildet als eine jeweilige Trajektorie eines Kontaktpunktes zwischen dem Inlay als der Tibia-Lagerfläche und der Femur-Lagerfläche;

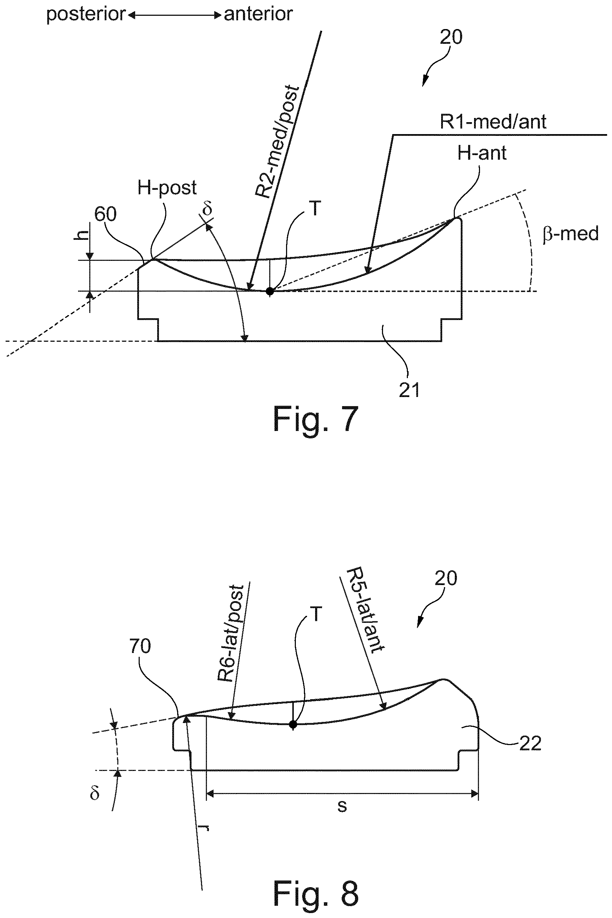

Fig. 7 ist, unter Bezugnahme auf Fig. 5, eine Schnittansicht des medialen Querschnitts des Inlays der Knieendoprothose gemäß dem Ausführungsbeispiel, insb. zur Veranschaulichung einer Tibia-seitigen Krümmungslinie der medialen Lagerschale in posterior-anteriorer Richtung;
Fig. 8 ist, unter Bezugnahme auf Fig. 5, eine Schnittansicht des lateralen Querschnitts des Inlays der Knieendoprothose gemäß dem Ausführungsbeispiel, insb. zur Veranschaulichung einer Krümmungslinie der lateralen Lagerschale in posterior-anteriorer Richtung;

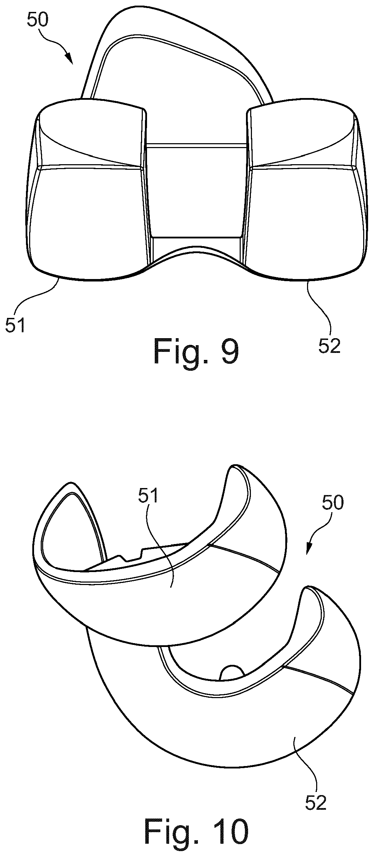

Fig. 9 ist eine erste (leicht) perspektivische Ansicht eines Femur-Teils gemäß dem Ausführungsbeispiel der Knieendoprothose nach der vorliegenden Offenbarung, gesehen von einem posterioren Blickpunkt, insb. zur Veranschaulichung einer, mit einer medialen Femur-Kondylen-Lagerfläche und einer lateralen Femur-Kondylen-Lagerfläche symmetrisch ausgebildeten, Femur-Lagerfläche;
Fig. 10 ist eine zweite perspektivische Ansicht eines Femur-Teils gemäß dem Ausführungsbeispiel, gesehen von einem schräg distalen Blickpunkt, in einer Blickrichtung auf die nach distal ausgerichtete Femur-Lagerfläche (bzw. gegen die Unterseite des Femur-Teils);

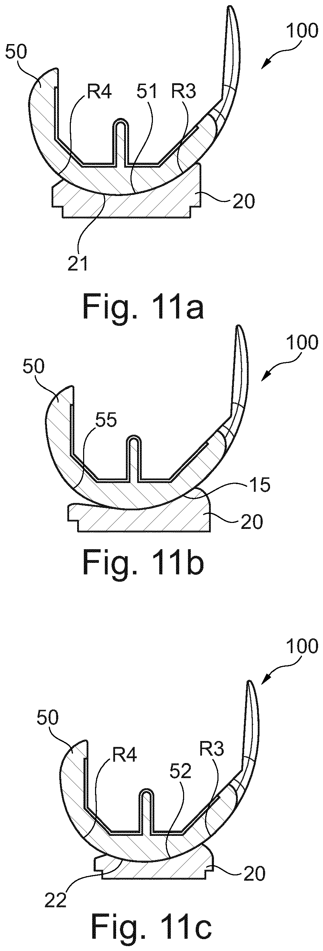

Figuren 11 a bis 11 c sind, unter Bezugnahme auf Fig. 5, jeweilige Schnittansichten der Knieendoprothese gemäß dem Ausführungsbeispiel für jeweils den medialen Querschnitt, den zentralen Querschnitt sowie den lateralen Querschnitt, insb. zur Veranschaulichung einer Kongruenz der Tibia-seitigen Krümmungslinie des Inlays zu einer Femur-seitigen Krümmungslinie des Femur-Teils, entlang der posterior-anterioren Richtung, bei einer gestreckten Knieendoprothese (d.h.bei einem Flexions-Winkel von ca. null Winkelgraden);
Figuren 12 a bis 12 d, in Gegenüberstellung zu den Figuren 13 a bis 13 d für den Stand der Technik, zeigen jeweilige Schnittansichten der Knieendoprothese gemäß dem Ausführungsbeispiel, entlang der posterior-anterioren Richtung, für vier jeweilige Flexions-Winkel bei ca. 0° (null Winkelgraden, gestreckt), bei ca. 30°, bei ca. 60° bzw. bei ca. 90° Flexion, zur Veranschaulichung der offenbarungsgemäßen Bewegungsentwicklung (insb. hinsichtlich einer quasisymmetrischen Femur-Lagerfläche, weiter hinsichtlich einer besonders bevorzugten Multi-Radien-Ausführungsvariante der Tibia-seitigen Krümmungslinie);
Figuren 13 a bis 13 d zeigen jeweilige Schnittansichten einer Knieendoprothese gemäß dem Stand der Technik, entlang der posterior-anterioren Richtung, für vier jeweilige Flexions-Winkel bei ca. 0° (null Winkelgraden, gestreckt), bei ca. 30°, bei ca. 60° bzw. bei ca. 90° Flexion, zur Veranschaulichung der Bewegungsentwicklung einer vorbekannten medial stabilen Knienendoprothese bzw. einer medialer-Drehpunkt-Konstruktion (insb. hinsichtlich einer asymmetrischen Femur-Lagerfläche und einer einen konstanten Radius aufweisenden Tibia-seitigen Krümmungslinie);

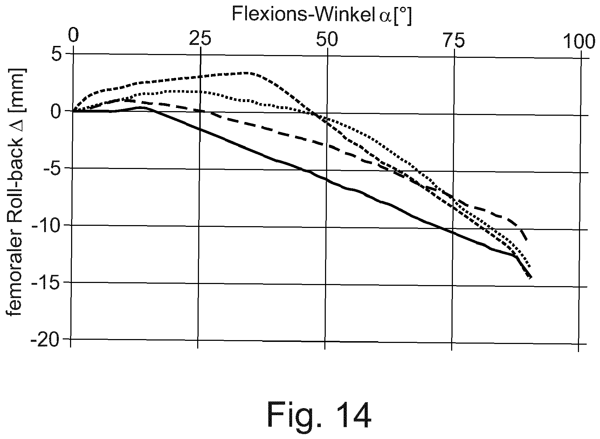

Fig. 14 zeigt ein Kennfeld mit vier kinematischen Kennlinien zur Veranschaulichung des Einflusses verschiedener Formausgestaltungen von einer Tibia-Lagerfläche und einer Femur-Lagerfläche zur gemeinsamen Ausbildung einer jeweiligen Gleitlagerung, nämlich zum Vergleich eines weiteren bevorzugten Ausführungsbeispiels einer Knieendoprothese nach der vorliegenden Offenbarung versus drei vorbekannten Knieendoprothesen aus dem Stand der Technik (bzw. im Markt befindlichen Produkten von drei Herstellern), gezeigt als jeweilige Abhängigkeiten des femoralen "Roll-Backs" in einem Bereich von ca. 0° bis ca. 90° Flexions-Winkel, wie mittels kinematischer Finite-Elemente-Simulation bestimmt.

### Beschreibung der Ausführungsbeispiele

Fig. 1 zeigt eine perspektivische schematische Darstellung einer offenbarungsgemäßen Knieendoprothese 100 zur Veranschaulichung einer gesamten Anordnung für eine Gesamtknie-Arthroplastie, wie von einem postero-lateralen Blickpunkt gesehen:
Ein Femur-Teil 50 (bzw. die Oberschenkel- oder Femurkomponente) als ein proximales (oberes) Teil der offenbarungsgemäßen Knieendoprothese 100 kann vorzugsweise mit zwei kondylenförmigen Halbschalen zur Ausbildung einer Femur-Lagerfläche vorgesehen sein. Unter Verweis auf die das Femur-Teil 50 für sich genommen darstellenden Figuren 9 und 10 sei hier vorweggenommen, dass die Femur-Lagerfläche eine konvexe mediale Femur-Kondylen-Lagerfläche 51 und eine konvexe laterale Femur-Kondylen-Lagerfläche 52 aufweist. Dabei ist laterale Femur-Kondylen-Lagerfläche 52 (im Wesentlichen) gleich bzw. symmetrisch zu der medialen Femur-Kondylen-Lagerfläche 51 ausgebildet.

Wie in Fig.1 schematisch angedeutet, ist das Femur-Teil 100 eingerichtet, um von einem Operateur am distalen Ende des Femurs (an den Kondylen bzw. Gelenkknorren) nach Entfernung der geschädigten Gelenkflächen aufgesetzt zu werden. Beispielsweise kann das Femur-Teil 100 mit den beiden (in der Darstellung der Figur 1 vertikal hochstehenden), nur exemplarisch abgebildeten, Gelenksstiften im Femur-Knochen in Richtung proximal verankert werden.

Figur 1 veranschaulicht auch die Verankerung der Knieendoprothese 100 an der distalen Seite (in der Darstellung der Figur 1: an der Unterseite) eines das Femur-Teil gleitverschiebbar bzw. gleitlagernd aufnehmenden Tibia-Teils 10. So befindet sich an dem Tibia-Teil 10 ein nur exemplarisch abgebildeter kurzer Schaft 30, der im Unterschenkelknochen in Richtung nach distal verankert werden kann. Vorzugsweise kann der Schaft 30 bzw. die distale Verankerungsstruktur des Tibia-Teils 10 aus einer Metall-Legierung und/oder Keramik und/oder aus einem, vorzugsweise thermoplastischen und/oder duroplastischen, Polymeren, , bspw. aus Polyethylen ("All-Poly"-Tibia) und/oder Hochleistungspolymeren (z.B.: PEEK), bestehen.

Das Tibia-Teil 10 (bzw. die Schienbein-, Unterschenkel- oder Tibiakomponente) als ein distales (unteres) Teil der offenbarungsgemäßen Knieendoprothese 100 kann vorzugsweise in Art eine flachen Scheibe bzw. plateauförmigen Stützstruktur ausgebildet sein. Das Tibia-Teil 10 ist im Sinne einer Gegenlagerkomponente zu dem Femur-Teil 10 vorgesehen, um als eine Tibia-Lagerfläche eine Gelenkfläche einer natürlichen Tibia zu ersetzen.

Insbesondere kann (unter Verweis auf die folgenden Figuren 2 bis 8) die Tibia-Lagerfläche 11 als eine proximale Fläche eines an dem Tibia-Teil 10 proximal angeordneten und/oder anordbaren Inlays 20 ausgebildet sein. Dies sei im Folgenden anhand der diesbezüglichen Darstellungen für das Inlay 20 nach einem bevorzugten Ausführungsbeispiel der vorliegenden Offenbarung ausführlich beschrieben.

Figuren 2 bis 8 zeigen unterschiedliche Ansichten des Inlays 20 der medial stabilisierenden Knieendoprothese 100 gemäß dem Ausführungsbeispiel nach der vorliegenden Offenbarung. So zeigen Figuren 2 und 3 perspektivische Aufsichten, gesehen von einem postero-lateralen Blickpunkt (Fig. 2) bzw. von einem antero-medialen Blickpunkt (Fig. 3). In einer Blickrichtung auf eine nach proximal ausgerichtete Tibia-Lagerfläche 11 (d.h. bei einem aufrecht stehenden Patienten: auf die Oberseite der Tibia bzw. des Tibia-Teils 10 aus Fig. 1) sind darin eine, im Wesentliche konkave, mediale Lagerschale 21 und eine, im Wesentlichen konkave, laterale Lagerschale 22 zu erkennen. Auf diesen können die jeweils zugehörigen konvexe mediale Femur-Kondylen-Lagerfläche 51 und konvexe laterale Femur-Kondylen-Lagerfläche 52 (vgl. Figuren 9 und 10) verschiebbar gleitgelagert werden. Dabei liegen die mediale Lagerschale 21 und die laterale Lagerschale 22 nebeneinander angeordnet, entlang einer Transversalachse.

Die mediale Lagerschale 21, welche einen medialen Bereich der Tibia-Lagerfläche 15 definiert, und die laterale Lagerschale 22, welche einen lateralen Bereich der Tibia-Lagerfläche 15 definiert, schließen zwischen ihnen einen zentralen Bereich der Tibia-Lagerfläche 15 ein (vgl. in Fig. 5 die Bezeichnung einer medialen, zentralen bzw. lateralen sagittalen Schnittebene). Dabei ist die Tibia-Lagerfläche 15 im zentralen Bereich verengt. Weiter ist die Tibia-Lagerfläche in leichter U-Form bzw. in Nierenschalen-Form, mit nach posterior weisender Öffnung der Form, geformt.

Der zentrale Bereich geht anterior in eine Ausbuchtung bzw. einen Ausschnitt, eine Auskrümmung über, welche als eine anteriore Patella-Ausbuchtung 25 (Figuren 2, 3 und 6) dient, um der räumlichen Bewegung einer Patella(sehne) Platz zu schaffen. Dazu ist eine um die Tibia-Lagerfläche 15 umlaufende (proximale) Höhenkontur anterozentral (im Wesentlichen) konkav ausgebildet. Dabei entspricht die Patella-Ausbuchtung 25 hinsichtlich der Größe der konkaven Auskrümmung einem vorbestimmten Krümmungsradius einer individuellen und/oder typischen Patella(sehne). Wie insb. der Draufsicht in der Fig. 6 zu entnehmen, ist die Patella-Ausbuchtung 25 asymmetrisch ausgebildet. Dies dient dazu, die Ausbildung eines an der medialen Lagerschale 21 proximal stärker als an der lateralen Lagerschale 22 erhöhten Höhenkonturabschnittes zu ermöglichen. Insofern ist die Patella-Ausbuchtung asymmetrisch ausgebildet, um im zentralen Bereich der Tibia-Lagerschale 15 einen zum antero-medialen Gipfelpunkt P steileren Anstieg des anterioren Höhenkonturabschnittes auszubilden.

Wie insb. den Figuren 2 und 3 mit ihrer perspektivischen Darstellungsform weiter zu entnehmen ist, sind die mediale Lagerschale 21 und die laterale Lagerschale hinsichtlich ihrer dreidimensionalen Hüllkontur, insb. hinsichtlich ihrer proximalen Höhenkontur (erkennbar als außen umlaufende durchgezogene Körperkante), unterschiedlich (dreidimensional) ausgeformt. So ist die mediale Lagerschale 21 insgesamt stärker nach proximal (bzw. oben) ausgeprägt. Insofern ist die mediale Lagerschale 21 an ihrer anterioren Seite bzw. in einem antero-medialen Höhenkonturabschnitt des anterioren Höhenkonturabschnitts zur Ausbildung einer anterioren Lippe hochgezogen, um in einem antero-medialen Gipfelpunkt P zu gipfeln. Dabei fällt der antero-mediale Gipfelpunkt P mit zumindest einem am meisten nach proximal erhöhten (bzw. gegenüber einer Transversalebene höchsten) Punkt zusammen. Diese bevorzugte Formgebung bewirkt, dass die Gleitlagerführung auf der medialen Seite stärker ausfällt als auf der lateralen Seite.

Somit bilden die mediale Lagerschale 21 und die laterale Lagerschale 22 zueinander eine asymmetrische Tibia-Lagerfläche 15 aus. Insbesondere ist die Asymmetrie auf eine zwischen ihnen liegende, d.h. zentrale, Sagittalebene bezogen bzw. ausgebildet.

Wie insbesondere anhand der Bezugszeichen in der eine Rückansicht (von posterior) des Inlays 20 zeigenden Figur 5 zu entnehmen, weist die um die asymmetrische Tibia-Lagerfläche 15 mit der medialen Lagerschale 21 und der lateralen Lagerschale 22 außen umlaufende proximale Höhenkontur aus. Auf einem anterioren Höhenkonturabschnitt der Höhenkontur (in Figur 5 hintere Konturlinie) liegt eine Vielzahl anteriorer Höhenpunkt H-ant. Auf einem posterioren Höhenkonturabschnitt der Höhenkontur (in Figur 5 vordere Konturlinie) liegt eine Vielzahl posteriorer Höhenpunkte H-post. Wie in Figur 5 gezeigt, liegen alle anterioren Höhenpunkt H-ant gegenüber allen posterioren Höhenpunkten H-post proximal erhöht bzw. höher. Insbesondere trifft dies für die mediale und die zentrale und die laterale Sagittalebene zu, welche anhand von strichpunktierten Linien als die jeweiligen Schnittebenen gekennzeichnet sind. Die zugehörigen Querschnittsansichten sind in den Ansichten der Figuren 7 und 11a (medialer Schnitt); der Figur 11b (zentraler Schnitt); sowie der Figuren 8 und 11c (lateraler Schnitt) dargestellt. Mit anderen Worten, ist der gesamte anteriore Höhenkonturabschnitt gegenüber dem gesamten posterioren Höhenkonturabschnitt (nach) proximal erhöht. Anders herum ausgedrückt, ist der gesamte posteriore Höhenkonturabschnitt gegenüber dem gesamten anterioren Höhenkonturabschnitt (nach) distal abgesenkt. Dies zeigt sich auch in dem Umstand, dass in der in Figur 4 gezeigten Vorderansicht des Inlays 20 der Knieendoprothose 100 gemäß dem Ausführungsbeispiel die Sicht auf den gesamten posterioren Höhenkonturabschnitt hinter dem anterioren Höhenkonturabschnitt verdeckt bleibt. Dabei bildet der antero-mediale Gipfelpunkt P den zumindest einen am meisten nach proximal erhöhten (bzw. gegenüber einer Transversalebene höchsten) Höhenpunkt H-ant auf dem anterioren Höhenkonturabschnitt aus.

Insbesondere Fig. 6, welche eine Draufsicht des Inlays 20, gesehen von einem proximalen Blickpunkt, in einer Blickrichtung nach distal auf das Inlay (bzw. auf die Oberseite des Tibia-Teils), dient der Veranschaulichung von drei jeweilig unabhängig bevorzugten Ausführungsbeispielen bzw. Varianten a, b, c einer jeweiligen Leitkrümmungslinie. Dabei wird als Leitkrümmungslinie (der Gleitlagerung) diejenige Linie bezeichnet, welche eine jeweilige Trajektorie eines Kontaktpunktes zwischen dem die Tibia-Lagerfläche 15 ausbildenden (Ober-)Fläche des Inlays 20 und der Femur-Lagerfläche 55 abbildet. Die Variante b bezeichnet eine erste Leitkrümmungslinie (stärker-kurvig), welche im Vergleich zu den Varianten einer zweiten Leitkrümmungslinie a (leicht-kurvig) und einer dritten Leitkrümmungslinie c (in etwa gerade) mit dem stärksten Krümmungsradius ausgeprägt ist. Die erste Leitkrümmungslinie b repräsentiert eine offenbarungsgemäß bewirkte Gleitlagerung bzw. Führung der Kinematik, bei welcher die Trajektorie entlang ihres Verlaufs nach posterior eine stärkere Einbiegung hin zu zentral erfährt. Hinsichtlich der medialen ersten Leitkrümmungslinie b erfährt die zugehörige Trajektorie entlang ihres Verlaufs aus einem mittigen Bereich der medialen Lagerschale 21 heraus nach posterior eine stärkere Einbiegung hin in Richtung nach zentral bzw. lateral.

Wie bereits besprochen, besteht auf der medialen Seite eine Tendenz, mehr oder weniger ortsfest zu bleiben, während auf der lateralen Seite Ausweichbewegungen nach rückwärts bzw. posterior bewirkt werden. Damit wird, da der Abstand A-Inlay (siehe Figur 5) zwischen der medialen Lagerschale 21 und der lateralen Lagerschale 22 festgelegt ist, der laterale Kontaktpunkt sich rückwärts und ein wenig nach innen zum zentralen Bereich des Inlays 20 hinbewegen bzw. nach lateral abbiegen.

Es hat sich (im Sinne eines kumulativen oder alternativen Merkmals zu den in den Ansprüchen beanspruchten technischen Merkmalen) herausgestellt, dass insbesondere diejenige Leitkrümmungslinie eine optimale Trajektorie beschreibt, welche (zumindest in einem posterioren Trajektorienabschnitt) kreisförmig mit einem Krümmungsradius von 76 bis 78 mm, insbesondere von ca. 77 mm, aus einem Zentrum der Tibia-Lagerfläche 19 gezogen wird. Es hat sich gezeigt, dass der vorgenannte besonders bevorzugte Krümmungsradius gegenüber einem größeren oder kleineren Krümmungsradius die Beweglichkeit der offenbarungsgemäßen Knieendoprothese optimiert und deren Verschleiss minimiert. Ferner wird aufgrund dessen die laterale Kongruenz ausgeglichen. Ein weiterer Vorteil zeigt sich darin, dass es dem Knie ermöglicht wird, zurückzurollen (vgl. Fig. Kennfeld der Fig. 14). Noch weiter fällt hierbei die Möglichkeit des Knies zu Drehbewegungen (Rotation) bei gleichzeitig maximaler Kongruenz der Gleitlagerung positiv ins Gewicht.

Figuren 7 bzw. 8 zeigen, unter Bezugnahme auf Fig. 5, jeweils eine sagittale Schnittansicht eines medialen Querschnitts des Inlays 20 bzw. eines lateralen Querschnitts des Inlays 20. Aus Fig. 7 geht eine (Tibia-seitige) Krümmungslinie der medialen Lagerschale 21 in posterior-anteriorer Richtung hervor. Hingegen aus Fig. 8 geht eine Krümmungslinie der lateralen Lagerschale 22 in posterior-anteriorer Richtung hervor.

In Fig. 7 ist zu erkennen, wie sich eine anteriore bzw. eine posteriore Lagerhöhe h jeweilig in analoger Weise definieren, nämlich im Sinne der Tiefe der medialen Lagerschale 21 (bzw. der Tiefe der lateralen Lagerschale 22), wenn von dem anterioren Höhenpunkt H-ant bzw. von dem posterioren Höhenpunkt H-post als einer oberen (proximalen) Lagerschalenkante bemessen.

Der posteriore Höhenpunkt H-post (s. Fig. 5), welcher in der medialen Sagittalebene der Fig. 7 liegt, bildet eine posteriore Lagerhöhe h aus. Dabei ist die posteriore Lagerhöhe auf einen proximal tiefsten (d.h. proximal am wenigsten erhöhten) Lager-Tiefpunkt T in der konkaven medialen Lagerschale 21 bezogen. Der anteriore Höhenpunkt H-ant (s. Fig. 5), welcher in der medialen Sagittalebene der Fig. 7 liegt, bildet eine anteriore Lagerhöhe h (ohne Bezugszeichen) aus. Dabei ist die anteriore Lagerhöhe, gleichfalls wie bereits die posteriore Lagerhöhe h, auf den Lager-Tiefpunkt T bezogen. Entsprechend dem bereits anhand der Figuren 4 und 5 besprochenen Merkmal, dass der anteriore Höhenkunturabschnitt, insb. der medialen Lagerschale 21, höher (proximaler) gezogen ist, als der posteriore Höhenkonturabschnitt, fällt die anteriore Lagerhöhe größer als die posteriore Lagerhöhe h aus. Weiter ist die maximale anteriore Lagerhöhe an dem antero-medialen Gipfelpunkt P (vgl. Fig. 5) ausgebildet.

Wie in Fig. 7 für deren mediale Sagittalebene betrachtet, spannt in der medialen Lagerschale 21 die anteriore Lagerhöhe ein Steigungsdreieck von dem anterioren Höhenpunkt H-ant zu dem proximal tiefsten Lager-Tiefpunkt T (als einem Scheitelpunkt des Steigungsdreieckes) auf. Dabei bildet das Steigungsdreieck einen an dem Lager-Tiefpunkt angeordneten medialen Steigungswinkel β-med aus. Der in der Fig. 7 vorgesehene mediale Steigungswinkel β-med bemisst ca. 22 Grad. Somit wird die gewünschte antero-mediale Stabilisierung im künstlichen Kniegelenk optimal weiter unterstützt.

Wie in Fig. 7 (mediale Sagittalebene) betrachtet, weist die mediale Lagerschale 21 einen ersten anterioren Flächenabschnitt auf, welcher mit einem antero-medialen Tibia-Lagerflächen-Radius R1-med/ant als einem ersten Radius konkav gekrümmt ist. Zudem weist die mediale Lagerschale 21 einen posterioren Flächenabschnitt auf, welcher mit einem postero-medialen Tibia-Lagerflächen-Radius R2-med/post als einem zweiten Radius dazu unterschiedlich konkav gekrümmt ist. Dabei ist der postero-mediale Tibia-Lagerflächen-Radius R2-med/post kleiner als der antero-mediale Tibia-Lagerflächen-Radius R1-med/ant.

Der antero-mediale Tibia-Lagerflächen-Radius R1-med/ant entspricht einem anterioren Femur-Kondylen-Radius R3 des Femur-Teils (siehe Fig. 11a). Dabei kann der anteriore Femur-Kondylen-Radius R3 vorzugsweise gemäß dem Femurradius eines natürlichen bzw. repräsentativen Femur-Kondylus gemäß einer spezifischen Femur-Größenklasse gewählt sein.

Der postero-mediale Tibia-Lagerflächen-Radius R2-med/post entspricht einem posterioren Femur-Kondylen-Radius R4 des Femur-Teils (siehe Fig. 11a). Dabei kann der posteriore Femur-Kondylen-Radius R4 vorzugsweise gemäß dem Femurradius eines natürlichen bzw. repräsentativen Femur-Kondylus gemäß einer spezifischen Femur-Größenklasse gewählt sein.

Wie in Fig. 8 (laterale Sagittalebene) gezeigt, weist die laterale Lagerschale 22 einen anterioren Flächenabschnitt auf, welcher mit einem antero-lateralen Tibia-Lagerflächen-Radius R5-lat/ant als einem fünften Radius konkav gekrümmt ist. Zudem weist die laterale Lagerschale 22 einen posterioren Flächenabschnitt auf, welcher mit einem postero-lateralen Tibia-Lagerflächen-Radius R6-lat/post als einem sechsten Radius dazu unterschiedlich konkav gekrümmt ist. Dabei ist der postero-laterale Tibia-Lagerflächen-Radius R6-lat/post größer als der antero-laterale Tibia-Lagerflächen-Radius R5-latlant.

Der antero-laterale Tibia-Lagerflächen-Radius R5-lat/ant entspricht dem anterioren Femur-Kondylen-Radius R3 des Femur-Teils (siehe Fig. 11c).

Der postero-laterale Tibia-Lagerflächen-Radius R6-lat/post im Vergleich zu dem posterioren Femur-Kondylen-Radius R4 (siehe Fig. 11c) größer ausgebildet, um offenbarungsgemäß eine postero-lateral verringerte Kongruenz auszubilden. Dazu beträgt der Tibia-Lagerflächen-Radius R6-lat/post 80 mm.

Wie Fig. 7 ferner zeigt, ist an der medialen Lagerschale 21 eine von dem posterioren Höhenpunkt H-post schräg nach distal verlaufende postero-mediale Phase 60 vorgesehen. Die postero-mediale Phase 60 dient der peripheren Materialwegnahme von der medialen Lagerschale 21, um einem potential bei starker Flexion posteromedial auftretendem Konflikt des Knochens mit der medialen Lagerschale 21 zu begegnen. In der Sagittalebene des Blattes der Fig. 7 steht die postero-mediale Phase 60 unter einem posterioren Phasenwinkel δ zu einer Transversalebene (bspw. einer flachen Bodenplatte des Inlays 20). Die postero-mediale Phase 60 ist mit einem posterioren Phasenwinkel δ von ca. 35 Grad angeschrägt.

Wie Fig. 8 ferner zeigt, ist an der lateralen Lagerschale 22 eine an dem posterioren Höhenpunkt H-post schräg nach distal verlaufende postero-laterale Phase 10. Wie bereits die postero-mediale Phase 60 dient auch die postero-laterale Phase 70 zur peripheren Materialwegnahme. In der Sagittalebene des Blattes der Fig. 8 steht die postero-laterale Phase 70 unter einem posterioren Phasenwinkel δ zu einer Transversalebene. Dabei bemisst der posteriore Phasenwinkel δ ca. 10 Grad. Ferner ist die postero-laterale Phase 70 mit einem postero-lateralen Rundungsradius r von ca. 12 mm abgerundet. Insofern verkürzt die postero-laterale Phase 70 den kongruenten Teil des posterioren Flächenabschnitts mit dem postero-lateralen Tibia-Lagerflächen-Radius R6-lat/post, welcher als Strecken-Lagerabschnitt s bezeichnet ist. Der Strecken-Lagerabschnitt s beträgt 90% der lichten Breite des Inlays 20 entlang einer Sagittalachse, ab der anterioren Körperkante des Inlays 20 gemessen.

Figuren 9 und 10 zeigen eine erste und eine zweite perspektivische Ansicht des Femur-Teils 50 gemäß dem Ausführungsbeispiel der Knieendoprothose nach der vorliegenden Offenbarung. Dabei ist Fig. 9 von einem posterioren Blickpunkt gesehen. Fig. 10 zeigt die zu der Fig. 9 im Raum gedrehte Perspektive, wenn von einem schräg distalen Blickpunkt gesehen und in einer Blickrichtung auf die nach distal ausgerichtete Femur-Lagerfläche bzw. gegen die Unterseite des Femur-Teils 50. Aus den Figuren 9 und 10 geht hervor, wie bereits im Zusammenhang der Fig. 1 beschrieben, dass die mediale Femur-Kondylen-Lagerfläche 51 und die laterale Femur-Kondylen-Lagerfläche 52 als zwei (im Wesentlichen) gleich gestaltete Bereiche der Femur-Lagerfläche diese symmetrisch ausbilden.

Wie aus insbesondere der Figur 9 weiterhin hervorgeht, ist die Formgebung im anterioren kappenförmigen Abschnitt des Femur-Teils 50, welcher als abgerundetes Dreieck anmutet, für die Beurteilung des Merkmals einer nicht-asymmetrischen, insbesondere einer hinsichtlich einer Sagittalebene spiegelsymmetrischen, Femur-Lagerfläche unerheblich. Insofern ist auf den Vergleich der als Femur-Lagerfläche fungierenden medialen Femur-Kondylen-Lagerfläche 51 und der lateralen Femur-Kondylen-Lagerfläche 52 abzustellen. Sprich, es sind diejenigen Flächenbereiche als Femur-Lagerfläche relevant, welche eine (funktional intendierte) Kontaktzone mit der zur Ausbildung der verschiebbaren Gleitlagerung vorgesehenen Tibia-Lagerfläche (bei regulärer Flexion) ausbilden. Details zur Thematik der Kontaktzone bzw. Kongruenz geht es den im Folgenden besprochenen Figuren 11 a bis 11 c hervor.

Figuren 11 a bis 11 c zeigen jeweilige Schnittansichten der Knieendoprothese gemäß dem Ausführungsbeispiel, entlang der posterior-anterioren Richtung (d.h. in einer Sagittalebene, posterior jeweils links in Fig. 11). Und zwar betreffen diese, unter Bezugnahme auf die in der Fig. 5 angezeichneten Schnittlinien, den medialen Querschnitt (Fig. 11a), den zentralen Querschnitt (Fig. 11b) sowie den lateralen Querschnitt (Fig. 11c). Dabei ist die Situation bei einer gestreckten Knieendoprothese (d.h. bei einem Flexions-Winkel von ca. null Winkelgraden) dargestellt.

Es sind die drei hier gezeigten Querschnitte hinsichtlich der Ausbildung der verschiebbaren Gleitlagerung aus dem auf dem Inlay 20 gelagerten Femur-Teil 50 relevant. So geht hieraus die hohe Kongruenz der Tibia-seitigen Krümmungslinie des Inlays 20 (wie vorstehend anhand der Figur 7 für den medialen Querschnitt und anhand der Figur 8 für den lateralen Querschnitt besprochen) zu einer Femur-seitigen Krümmungslinie des Femur-Teils 50 hervor.

Wie Fig. 11a (mediale Sagittalebene) zeigt, weist die mediale Femur-Kondylen-Lagerfläche 51 einen anterioren Flächenabschnitt auf, welcher mit einem anterioren Femur-Kondylen-Radius R3 als einem dritten Radius konvex gekrümmt ist. Sagittal gegenüberliegend weist die mediale Femur-Kondylen-Lagerfläche 51 einen posterioren Flächenabschnitt auf, welcher mit einem posterioren Femur-Kondylen-Radius R4 als einem vierten Radius konvex gekrümmt ist.

In den Figuren 11a bis 11c ist das Femur-Teil 50 mit dem anterioren Femur-Kondylen-Radius R3 und dem posteriore Femur-Kondylen-Radius R4 in bevorzugter Weise derart ausgebildet, dass diese passend für eine spezifische Femur-Größenklasse für einen Patienten vorbestimmt sind. Damit entspricht der anteriore Femur-Kondylen-Radius R3 dem anterioren Femurradius eines natürlichen bzw. repräsentativen Femur-Kondylus gemäß der spezifischen Femur-Größenklasse, während der posteriore Femur-Kondylen-Radius R4 dem posterioren Femurradius gemäß der spezifischen Femur-Größenklasse entspricht.

In der in der Fig. 11a gezeigten Extensionsstellung (0 Grad Flexions-Winkel) besteht eine hohe Kongruenz, insb. Spielpassung, der medialen Lagerschale 21 des Inlays 20, wie im Detail anhand der Fig. 7 besprochen, zu dem gleitlagernd auf der medialen Lagerschale 21 aufliegenden Femur-Teil 50.

Wie Figur 11c (laterale Sagittalebene) zeigt, weist die laterale Femur-Kondylen-Lagerfläche 52 einen anterioren bzw. posterioren Flächenabschnitt auf, welcher - aufgrund der Symmetrie der Femur-Lagerfläche - gleichfalls mit dem anterioren Femur-Kondylen-Radius R3 (dritten Radius) bzw. mit dem posterioren Femur-Kondylen-Radius R4 (vierten Radius) gemäß der Femur-Größenklasse konvex gekrümmt ist. Dabei ist die laterale Femur-Kondylen-Lagerfläche 52 auf der im Detail anhand der Fig. 8 besprochenen lateralen Lagerschale 22 des Inlays 20 gleitgelagert. Der gezeigten Extensionsstellung (0 Grad Flexions-Winkel) ist die (insb. anteriore) Kongruenz zu entnehmen.

Figuren 12 a bis 12 d zu der vorliegenden Offenbarung zeigen, in Gegenüberstellung zu den den Stand der Technik zeigenden Figuren 13 a bis 13 d, jeweilige Schnittansichten der Knieendoprothese 100 gemäß dem Ausführungsbeispiel, entlang der posterior-anterioren Richtung. Dabei beinhaltet die Knieendoprothese 100 das Inlay 20 (siehe Figuren 2 bis 7) im Zusammenspiel mit dem Femur-Teil 50 (siehe Figuren 8 und 9). Aus der sequenziellen Darstellung lässt sich das kinematische Verhalten bei Kniebeugung für vier jeweilige Flexions-Winkel entnehmen, ausgehend von bei ca. 0° (Fig. 12a) entsprechend einem gestreckten Bein, über mittlere Flexion bei ca. 30° (Fig. 12b), bei ca. 60° (Fig. 12c) bis hin zu ca. 90° (Fig. 12d) Flexion entsprechend einem senkrecht abgewickelten Knie. Figuren 12 a bis 12 d veranschaulichen somit eine offenbarungsgemäße Bewegungsentwicklung, insb. hinsichtlich der Wirkung einer quasisymmetrischen Femur-Lagerfläche, weiter hinsichtlich einer besonders bevorzugten Multi-Radien-Ausführungsvariante der Tibia-seitigen Krümmungslinie. In der Abfolge der Figuren 12b bis 12d ist ein offenbarungsgemäßer Bewegungsspielraum B als jeweiliger Doppelpfeil bezeichnet. Daraus geht hervor, dass ein solcher über einen großen Anteil einer sagittalen Breite der Knieendoprothese 100 in positiver Weise vorliegt. Ferner ist eine besonders hohe Flexibilität im Sinne eines noch weiter erhöhten Bewegungsspielraum B bei ca. 90° Flexion zu entnehmen. Die hier dargestellten kinematischen Eigenschaften unterstützen sportliche Bewegungsabläufe.

Figuren 13 a bis 13 d zeigen in Analogie zu den vorstehend besprochenen Figuren 12 a bis 12 d entsprechende jeweilige Schnittansichten einer Knieendoprothese gemäß dem Stand der Technik. Figuren 13 a bis 13 d veranschaulichen also eine herkömmliche Bewegungsentwicklung einer vorbekannten medial stabilen Knienendoprothese bzw. einer medialer-Drehpunkt-Konstruktion. Es ist anhand des als jeweiliger Doppelpfeil bezeichneten Bewegungsspielraums B, welcher jeweilig bei ca. 30° (Fig. 13b), bei ca. 60° (Fig. 13c) bzw. bei ca. 90° (Fig. 13d) Flexions-Winkel bemaßt ist, zu entnehmen, dass dieser im Stand der Technik geringfügig bzw. eng begrenzt ausfällt. Dabei ist der herkömmlich enge Bewegungsspielraum in der medialer-Drehpunkt-Konstruktion mit einer asymmetrischen Femur-Lagerfläche und einer einen konstanten Radius aufweisenden Tibia-seitigen Krümmungslinie begründet.

Fig. 14 zeigt ein Kennfeld mit vier kinematischen Kennlinien zur vergleichsweisen Veranschaulichung des Einflusses verschiedener Formausgestaltungen von einer Tibia-Lagerfläche und einer Femur-Lagerfläche zur gemeinsamen Ausbildung einer jeweiligen Gleitlagerung. In dem Kennfeld ist der Einfluss des Flexion-Winkels α auf den femoralen Roll-Back Δ dargestellt. Dabei ist vorliegend der femorale Roll-back Δ in Form von negativen Werten (negativer Wertebereich) ausgewiesen, insofern dieser eine Rückwärtsbewegung betrifft. Sprich, ein im Kennfeld positiver y-Wert für den femoralen Roll-back weist umgekehrt eine Roll-front-Bewegung aus. Dabei wurden die jeweiligen Kennlinien in einem Bereich von ca. 0° bis ca. 90° Flexions-Winkel gleichermaßen mittels einer kinematischen Simulationsmethode auf Basis von Finite-Elemente-Berechnungen bestimmt. Dabei wurde jeweils eine Situation ohne hinteres Kreuzband simuliert.

Zum einen ist ein weiteres bevorzugtes Ausführungsbeispiel einer offenbarungsgemäßen Knieendoprothese nach der vorliegenden Offenbarung mittels der durchgezogenen Kennlinie dargestellt. Gegenübergestellt sind zum anderen drei Kennlinien für unterschiedliche vorbekannte Knieendoprothesen aus dem Stand der Technik, welche im Markt befindliche Produkte von drei Herstellern betreffen.

Figur 14 zeigt, dass bei allen drei vorbekannten Knieendoprothesen in der beginnenden Flexion unterschiedlich hohe Niveaus der Roll-front-Bewegung bewirkt werden, insofern sie bis ca. 30° bzw. ca. 45° Flexions-Winkel noch positive y-Werte aufweisen. Dabei zeigt die drei unterbrochenen Kennlinien die vorbekannten Knieendoprothesen, in Reihenfolge von oben nach unten in Fig. 14, wie folgt: Type "PFC Sigma" (von Hersteller J. & J), Type "Columbus" (von Aesculap) sowie Type "Attune" (von Depuy). Erst bei weiterem Anstieg des Flexions-Winkel darüberhinaus, kann bei den drei vorbekannten Knieendoprothesen der zum Nachempfinden sportlicher natürlicher Bewegungsabläufe erwünschte Roll-back (negative Werte) stattfinden.

Demgegenüber belegt für das weitere bevorzugte Ausführungsbeispiel nach der vorliegenden Offenbarung die durchgezogene Kennlinie, dass diese sich signifikant und in vorteilhafter Weise vom Stand der Technik abhebt. So ist ein minimales Auftreten von Roll-front (positiver Wertebereich) in der frühen Flexion zu verzeichnen, auch in der Situation ohne hinteres Kreuzband, sowie ein sehr gutes (lineares) Zurückrollen in der weiteren Flexion.

Wie in dem Kennfeld der Fig. 14 aufgetragen, wird in der Knieendoprothese des weiteren bevorzugten Ausführungsbeispiels ein femoraler Roll-back bewirkt. Der femorale Roll-back (erkennbar als Werte im negativen Bereich, da auf Roll-front-Achse, d.h. auf eine in Vorwärtsrichtung positive Koordinatenachse bezogen) beträgt hierin kleinergleich minus 1,5 mm bei 30 Grad bzw. kleinergleich minus 6 mm bei 60 Grad bzw. kleinergleich minus 11 mm bei ca. 90 Grad Flexions-Winkel. Entsprechend bedeutet dies, dass ein Betragswert des femoralen Roll-backs vorzugsweise größergleich 1,5 mm bei 30 Grad und/oder größergleich 6 mm bei 60 Grad und/oder größergleich 11 mm bei ca. 90 Grad Flexions-Winkel beträgt.

Ferner ist der offenbarungsgemäßen Kennlinie des weiteren bevorzugten Ausführungsbeispiels zu entnehmen, dass in einem Umkehrbereich bei ca. 14 Grad Flexions-Winkel die femorale Bewegung von einer (ohnehin minimalen) Roll-front in den (im Weiteren betragsmäßig zunehmenden) Roll-back wechselt. Bis zum Erreichen des Umkehrbereiches bei ca. 14 Grad Flexions-Winkel, d.h. in der geringen bzw. frühen Flexion, beträgt der femorale Roll-front kleinergleich ca. 0,5 mm.

Diese Ergebnisse aus dem Kennfeld der Fig. 14 bestätigen, dass eine vollständige Kongruenz nicht notwendig ist, um eine Stabilität in der frühen Flexion zu gewährleisten. Und durch das offenbarungsgemäße Abweichen von einer eine vollständige Kongruenz, kann vorteilhaft erreicht werden, dass diese nicht dem erwünschten femoralen Roll-back in der weiteren Flexion nachteilig entgegensteht bzw. diesen unterdrückt.

### Bezugszeichen

- 10: Tibia-Teil
- 15: Tibia-Lagerfläche
- 20: Inlay
- 21: mediale Lagerschale
- 22: laterale Lagerschale
- 25: Patella-Ausbuchtung
- 50: Femur-Teil
- 51: mediale Femur-Kondylen-Lagerfläche
- 52: laterale Femur-Kondylen-Lagerfläche
- 55: Femur-Lagerfläche
- ..60: postero-mediale Phase
- ..70: postero-laterale Phase
- 100: Knieendoprothese
- B: Bewegungsspielraum
- H-ant: anteriorer Höhenpunkt
- H-post: posteriorer Höhenpunkt
- P: antero-medialer Gipfelpunkt
- R1-med/ant: antero-medialer Tibia-Lagerflächen-Radius (erster Radius)
- R2-med/post: postero-medialer Tibia-Lagerflächen-Radius (zweiter Radius)
- R3: anteriorer Femur-Kondylen-Radius (dritter Radius)
- R4: posteriorer Femur-Kondylen-Radius (vierter Radius)
- R5-lat/ant: antero-lateraler Tibia-Lagerflächen-Radius (fünfter Radius)
- R6-lat/post: postero-lateraler Tibia-Lagerflächen-Radius (sechster Radius)
- T: Lager-Tiefpunkt
- a: zweite Leitkrümmungslinie
- b: erste Leitkrümmungslinie
- c: dritte Leitkrümmungslinie
- h: Lagerhöhe
- r: Rundungsradius
- s: Strecken-Lagerabschnitt
- β-med: medialer Steigungswinkel
- δ: posteriorer Phasenwinkel

## Patentansprüche

1. Medial stabilisierende Knieendoprothese (100) für eine Gesamtknie-Arthroplastie unter Erhalt oder unter Dissektion des hinteren Kreuzbandes mit:
einer Femur-Lagerfläche (55), welche an einem zur Festlegung an einem distalen Ende eines Femurs eingerichteten Femur-Teil (50) zur Ausrichtung nach distal vorgesehen ist; und
einer Tibia-Lagerfläche (15), welche an einem zur Festlegung an einem proximalen Ende einer Tibia eingerichteten Tibia-Teil (10) zur Ausrichtung nach proximal vorgesehen ist, wobei insbesondere die Tibia-Lagerfläche (15) als eine proximale Fläche eines an dem Tibia-Teil (10) proximal angeordneten und/oder anordbaren Inlays (20) ausgebildet ist,
wobei die Femur-Lagerfläche (55) eine konvexe mediale Femur-Kondylen-Lagerfläche (51) und eine konvexe laterale Femur-Kondylen-Lagerfläche (52) aufweist;
wobei die Tibia-Lagerfläche (15) eine konkave mediale Lagerschale (21) und eine konkave laterale Lagerschale (22) aufweist und zur Aufnahme und verschiebbaren Gleitlagerung der Femur-Lagerfläche (55) ohne einen festen medialen Drehpunkt der Femur-Lagerfläche (55) in der medialen Lagerschale (21) eingerichtet ist;
- wobei die mediale Lagerschale (21) und die laterale Lagerschale (22) zueinander eine asymmetrische Tibia-Lagerfläche (15) ausbilden; und
- die mediale Femur-Kondylen-Lagerfläche (51) und die laterale Femur-Kondylen-Lagerfläche (52) zueinander eine nicht-asymmetrische oder quasisymmetrische Femur-Lagerfläche (55) ausbilden,
**dadurch gekennzeichnet, dass**
- die mediale Femur-Kondylen-Lagerfläche (51) und die laterale Femur-Kondylen-Lagerfläche (52) zumindest in einem anterioren Femur-Kondylen-Radius (R3) jeweiliger anteriorer Flächenabschnitte übereinstimmen;
- die asymmetrische Tibia-Lagerfläche (15) eine außen umlaufende proximale Höhenkontur ausbildet;
- die Höhenkontur einen anterioren Höhenkonturabschnitt mit zumindest einem anterioren Höhenpunkt (H-ant) und einen posterioren Höhenkonturabschnitt mit zumindest einem posterioren Höhenpunkt (H-post) aufweist, wobei der anteriore Höhenpunkt (H-ant) gegenüber dem posterioren Höhenpunkt (H-post) hinsichtlich zumindest einer Sagittalebene proximal erhöht ist; und
- ein antero-medialer Höhenkonturabschnitt des anterioren Höhenkonturabschnitts an der medialen Lagerschale (21) einen proximal am meisten erhöhten anterioren Höhenpunkt (H-ant) als einen antero-medialen Gipfelpunkt (P) ausbildet.

2. Knieendoprothese (100) nach Anspruch 1, wobei:
der anteriore Höhenpunkt (H-ant) hinsichtlich einer Sagittalebene eine anteriore Lagerhöhe, in Bezug auf einen proximal tiefsten Lager-Tiefpunkt (T) in der konkaven medialen und/oder lateralen Lagerschale (21, 22), ausbildet; und
der posteriore Höhenpunkt (H-post) hinsichtlich einer Sagittalebene eine posteriore Lagerhöhe (h), in Bezug auf einen proximal tiefsten Lager-Tiefpunkt (T) in der konkaven medialen und/oder lateralen Lagerschale (21, 22), ausbildet;
**dadurch gekennzeichnet, dass** die anteriore Lagerhöhe größer als die posteriore Lagerhöhe (h) ist, weiter bevorzugt die maximale anteriore Lagerhöhe an dem antero-medialen Gipfelpunkt (P) ausgebildet ist.

3. Knieendoprothese (100) nach dem direkt vorhergehenden Anspruch, wobei:
- in einer medialen Sagittalebene ein Steigungsdreieck, welches von dem anterioren Höhenpunkt (H-ant) mit der anterioren Lagerhöhe zu dem proximal tiefsten Lager-Tiefpunkt (T) in der medialen Lagerschale (21) aufgespannt ist, einen an dem proximal tiefsten Lager-Tiefpunkt (T) angeordneten medialen Steigungswinkel (β-med) ausbildet;
**dadurch gekennzeichnet, dass** der mediale Steigungswinkel (β-med) 12 bis 32 Grad, weiter bevorzugt 17 bis 27 Grad, insbesondere ca. 22 Grad, bemisst.

4. Knieendoprothese (100) nach einem der vorhergehenden Ansprüche, wobei:
- ein distalster Punkt der medialen Femur-Kondylen-Lagerfläche (51) einen medialen Extensions-Lagerflächen-Kontaktpunkt, welcher mit der medialen Lagerschale (21) der Tibia-Lagerfläche (15) bei ca. 0 Grad Flexions-Winkel in Kontakt steht, ausbildet;
**dadurch gekennzeichnet, dass** der mediale Extensions-Lagerflächen-Kontaktpunkt,
- wenn bei ca. 0 Grad Flexions-Winkel hinsichtlich einer Sagittalebene als Winkelsegment auf eine anteriore Kante der medialen Femur-Kondylen-Lagerfläche (51) bezogen, bei 50 bis 70 Grad, weiter bevorzugt bei 58 bis 62 Grad, insbesondere bei ca. 60 Grad, angeordnet ist; und/oder
- wenn bei ca. 0 Grad Flexions-Winkel hinsichtlich einer Sagittalebene als Winkelsegment auf eine anteriore Kante der medialen Lagerschale (21) der Tibia-Lagerfläche (15) bezogen, bei 55 bis 75 Grad, weiter bevorzugt bei 63 bis 67 Grad, insbesondere bei ca. 65 Grad angeordnet ist.

5. Knieendoprothese (100) nach einem der vorhergehenden Ansprüche, **dadurch gekennzeichnet, dass**
- die konkave mediale Lagerschale (21) hinsichtlich einer medialen Sagittalebene zumindest einen anterioren Flächenabschnitt mit einem ersten Radius, welcher einen antero-medialen Tibia-Lagerflächen-Radius (R1-med/ant) ausbildet, und zumindest einen posterioren Flächenabschnitt mit einem zum ersten Radius unterschiedlichen, vorzugsweise kleineren, zweiten Radius, welcher einen postero-medialen Tibia-Lagerflächen-Radius (R2-med/post) ausbildet; wobei insbesondere:
- der antero-mediale Tibia-Lagerflächen-Radius (R1-med/ant) kongruent, insb. identisch, zu dem anterioren Femur-Kondylen-Radius (R3), welcher als ein dritter Radius des anterioren Flächenabschnitts der medialen Femur-Kondylen-Lagerfläche (51) bezeichnet ist, vorzugsweise gemäß einer Femur-Größenklasse für einen Patienten vorbestimmt ist, ausgebildet ist; und/oder
- der postero-mediale Tibia-Lagerflächen-Radius (R2-med/post) kongruent, insb. identisch, zu einem posterioren Femur-Kondylen-Radius (R4), welcher als ein vierter Radius eines posterioren Flächenabschnitts der medialen Femur-Kondylen-Lagerfläche (51) ausgebildet ist, vorzugsweise gemäß einer Femur-Größenklasse für einen Patienten vorbestimmt ist, ausgebildet ist.

6. Knieendoprothese (100) nach einem der vorhergehenden Ansprüche, **dadurch gekennzeichnet, dass**
- die konkave laterale Lagerschale (22) hinsichtlich einer lateralen Sagittalebene zumindest einen anterioren Flächenabschnitt mit einem fünften Radius, welcher einen antero-lateralen Tibia-Lagerflächen-Radius (R5-lat/ant) ausbildet, und zumindest einen posterioren Flächenabschnitt mit einem zum fünften Radius unterschiedlichen, vorzugsweise größeren, sechsten Radius, welcher einen postero-lateralen Tibia-Lagerflächen-Radius (R6-lat/post) ausbildet, ausbildet; wobei insbesondere, unter Bezugnahme auf den direkt vorhergehenden Anspruch:
- der antero-laterale Tibia-Lagerflächen-Radius (R5-lat/ant) kongruent, insb. identisch, zu dem anterioren Femur-Kondylen-Radius (R3) ausgebildet ist; und/oder
- der postero-laterale Tibia-Lagerflächen-Radius (R6-lat/post) größer als der posteriore Femur-Kondylen-Radius (R4) ausgebildet ist, weiter bevorzugt um zumindest 110 Prozent größer bemessen ist, insbesondere größergleich 80 mm beträgt.

7. Knieendoprothese (100) nach einem der Ansprüche 1 bis 6, wobei:
- an der medialen Lagerschale (21) eine von dem posterioren Höhenpunkt (H-post) schräg nach distal verlaufende postero-mediale Phase (60) zur peripheren Materialwegnahme vorgesehen ist, welche, wenn in einer Sagittalebene projiziert, unter einem posterioren Phasenwinkel (δ) zu einer Transversalebene steht;
**dadurch gekennzeichnet, dass** die postero-mediale Phase (60) mit einem posterioren Phasenwinkel (δ) von 30 bis 40 Grad, weiter bevorzugt von 33 bis 37 Grad, insbesondere von ca. 35 Grad, angeschrägt ist.

8. Knieendoprothese (100) nach einem der Ansprüche 1 bis 7, wobei:
- an der lateralen Lagerschale (22) ein an dem posterioren Höhenpunkt (H-post) schräg nach distal verlaufende postero-laterale Phase (70) zur peripheren Materialwegnahme vorgesehen ist, welche, wenn in einer Sagittalebene projiziert, unter einem posterioren Phasenwinkel (δ) zu einer Transversalebene steht;
**dadurch gekennzeichnet, dass** die postero-laterale Phase (70) mit einem posterioren Phasenwinkel (δ) von 5 bis 15 Grad, weiter bevorzugt von 8 bis 12 Grad, insbesondere von ca. 10 Grad, ausgebildet und/oder mit einem postero-lateralen Rundungsradius (r) von 10 bis 14 mm, weiter bevorzugt von 11,2 bis 12,8 mm, insbesondere von ca. 12 mm, abgerundet ist.

9. Knieendoprothese (100) nach einem der vorhergehenden Ansprüche, **dadurch gekennzeichnet, dass**
- die Tibia-Lagerfläche (15) eine, zentral zwischen der medialen Lagerschale (21) und der lateralen Lagerschale (22) vorgesehene, anteriore Patella-Ausbuchtung (25) mit einer konkaven äußeren Körperkontur ausbildet; und insbesondere die Patella-Ausbuchtung (25) asymmetrisch ausgebildet und/oder kongruent zu einer für einen Patienten vorbestimmten Patella-Größenklasse und/oder einem statistisch-anthropometrisch vorbestimmten 50-Perzentil-Wert für eine Patella-Größenverteilung hinsichtlich einer repräsentativen Bevölkerungspopulation ausgebildet ist.

10. Knieendoprothese (100) nach einem der vorhergehenden Ansprüche, **dadurch gekennzeichnet, dass** die mediale Femur-Kondylen-Lagerfläche (51) und die laterale Femur-Kondylen-Lagerfläche (52) zueinander eine hinsichtlich einer dazwischen angeordneten Sagittalebene, im Wesentlichen, spiegelsymmetrische Femur-Lagerfläche (55) ausbilden.

11. Knieendoprothese (100) nach einem der vorhergehenden Ansprüche, **dadurch gekennzeichnet, dass** die mediale Femur-Kondylen-Lagerfläche (51) zumindest in einem distal-medialen Flächenabschnitt einen medialen Femur-Kondylen-Lagerflächenradius, insbesondere zur Nachempfindung eines natürlichen medialen Femurkondylus-Radius, gleichbleibend aufweist; wobei die laterale Femur-Kondylen-Lagerfläche (52) zumindest in einem lateral-medialen Flächenabschnitt einen anderen lateralen Femur-Kondylen-Lagerflächenradius, insbesondere zur Nachempfindung eines natürlichen lateralen Femurkondylus-Radius, geichbleibend aufweist; und wobei der mediale Femur-Kondylen-Lagerflächenradius und der laterale Femur-Kondylen-Lagerflächenradius voneinander maximal um einen Verhältnisfaktor von 0,8 bis 1,2, vorzugsweise von 0,9 bis 1,1, insbesondere von 0,95 bis 1,05, geringfügig abweichen.

12. Knieendoprothese nach einem der vorhergehenden Ansprüche, **dadurch gekennzeichnet, dass** die Tibia-Lagerfläche (15) zur Aufnahme und verschiebbaren Gleitlagerung der Femur-Lagerfläche (55) entlang einer durch eine Vielzahl von Kontaktpunkten ausgebildeten Leitkrümmungslinie (a, b, c) eingerichtet ist, so dass bei einer flektierenden Beugung der Knieendoprothese (100), vorzugsweise unter Last eines Norm-Körpergewichts von 75kg, von 0 Grad auf ca. 90 Grad Flexions-Winkel:
- eine Amplitude von minus 0 mm bis minus 11 mm femoraler Roll-back bewirkt wird, wobei in bevorzugter Weise der femorale Roll-back, wenn auf eine in Vorwärtsrichtung positive Koordinatenachse bezogen, kleinergleich minus 1,5 mm bei 30 Grad und/oder kleinergleich minus 6 mm bei 60 Grad und/oder kleinergleich minus 11 mm bei ca. 90 Grad Flexions-Winkel beträgt.

13. Knieendoprothese nach einem der vorhergehenden Ansprüche, **dadurch gekennzeichnet, dass** die Tibia-Lagerfläche (15) zur Aufnahme und verschiebbaren Gleitlagerung der Femur-Lagerfläche (55) eingerichtet ist, so dass bei einer flektierenden Beugung der Knieendoprothese (100) unter einem Gangzyklus, insbesondere einem Bewegungsablauf von 0-15-0-60 Grad Flexions-Winkel und vorzugsweise bei einer Belastung im Gang mit einem Norm-Körpergewicht von 75kg:
- eine mediale Bewegung der medialen Femurkondyle um delta 1 mm bis 4 mm, vorzugsweise im Wesentlichen 2 mm bewirkt wird; und
- eine laterale Bewegung der lateralen Femurkondyle um delta 6 mm bis 11 mm, vorzugsweise im Wesentlichen 9 mm bewirkt wird, sodass
in bevorzugter Weise bei einer flektierenden Beugung der Knieendoprothese (100) unter einem Gangzyklus von 0-15-0-60 Grad Flexions-Winkel und vorzugsweise bei einer Belastung im Gang mit einem Norm-Körpergewicht von 75kg:
- eine Bewegung der medialen Femurkondyle, von 0 mm auf max. 2 mm anterior und wieder zurück von 2 mm auf 0 mm posterior bewegt bewirkt wird; und
- eine Bewegung der lateralen Femurkondyle um 5,5 mm anterior, um 9 mm posterior und wieder um 3,5 mm anterior bewirkt wird.

14. Inlay (20), welches an dem Tibia-Teil (10) einer Knieendoprothese (100) nach einem der vorhergehenden Ansprüche proximal angeordnet, insbesondere integral mit dem Tibia-Teil (10) ausgebildet, und/oder eingerichtet anordbar ist,
- wobei eine proximale Fläche des Inlays (20) die eine konkave mediale Lagerschale (21) und eine konkave laterale Lagerschale (22) aufweisende Tibia-Lagerfläche (15) ausbildet, welche zur Aufnahme und verschiebbaren Gleitlagerung der eine konvexe mediale Femur-Kondylen-Lagerfläche (51) und eine konvexe laterale Femur-Kondylen-Lagerfläche (52) aufweisenden Femur-Lagerfläche (55) ohne einen festen medialen Drehpunkt der Femur-Lagerfläche (55) in der medialen Lagerschale (21) eingerichtet ist;
- wobei die mediale Lagerschale (21) und die laterale Lagerschale (22) zueinander eine asymmetrische Tibia-Lagerfläche (15) ausbilden;
- die mediale Femur-Kondylen-Lagerfläche (51) und die laterale Femur-Kondylen-Lagerfläche (52) zueinander eine nicht-asymmetrische, insbesondere eine hinsichtlich einer dazwischen angeordneten Sagittalebene spiegelsymmetrische, Femur-Lagerfläche (55) ausbilden
**dadurch gekennzeichnet, dass**
- die mediale Femur-Kondylen-Lagerfläche (51) und die laterale Femur-Kondylen-Lagerfläche (52) zumindest in einem anterioren Femur-Kondylen-Radius (R3) jeweiliger anteriorer Flächenabschnitte übereinstimmen;
- die asymmetrische Tibia-Lagerfläche (15) eine außen umlaufende proximale Höhenkontur ausbildet;
- die Höhenkontur einen anterioren Höhenkonturabschnitt mit zumindest einem anterioren Höhenpunkt (H-ant) und einen posterioren Höhenkonturabschnitt mit zumindest einem posterioren Höhenpunkt (H-post) aufweist, wobei der anteriore Höhenpunkt (H-ant) gegenüber dem posterioren Höhenpunkt (H-post) hinsichtlich zumindest einer Sagittalebene proximal erhöht ist; und
- ein antero-medialer Höhenkonturabschnitt des anterioren Höhenkonturabschnitts an der medialen Lagerschale (21) einen proximal am meisten erhöhten anterioren Höhenpunkt (H-ant) als einen antero-medialen Gipfelpunkt (P) ausbildet.

## Claims

1. A medially stabilising knee endoprosthesis (100) for a total knee arthroplasty while retaining or dissecting the posterior cruciate ligament, the medially stabilising knee endoprosthesis comprising:
- a femur bearing surface (55), which is provided on a femur part (50) for alignment in the distal direction, said femur part being designed to be fixed to a distal end of a femur; and
- a tibia bearing surface (15), which is provided on a tibia part (10) for alignment in the proximal direction, said tibia part being designed to be fixed to a proximal end of a tibia, wherein in particular the tibia bearing surface (15) is formed as a proximal surface of an inlay (20) proximally arranged and/or arrangeable on the tibia part (10),
- wherein the femur bearing surface (55) comprises a convex medial femur-condyle bearing surface (51) and a convex lateral femur-condyle bearing surface (52),
- wherein the tibia bearing surface (15) comprises a concave medial bearing shell (21) and a concave lateral bearing shell (22) and is designed for receiving and displaceably slidably bearing the femur bearing surface (55), without a fixed medial center point of the femur bearing surface (55), in the medial bearing shell (21);
- wherein the medial bearing shell (21) and the lateral bearing shell (22) form an asymmetric tibia bearing surface (15) with respect to one another;
- and the medial femur-condyle bearing surface (51) and the lateral femur-condyle bearing surface (52) form a non-asymmetrical or quasi-symmetrical femur bearing surface (55) with respect to one another,
**characterized in that**
- the medial femur-condyle bearing surface (51) and the lateral femur-condyle bearing surface (52) correspond at least in an anterior femur-condyle radius (R3) of relevant anterior surface portions.
- the asymmetric tibia bearing surface (15) forms an external continuous proximal height contour;
- the height contour comprises an anterior height contour portion having at least one anterior height point (H-ant) and a posterior height contour portion having at least one posterior height point (H-post), wherein the anterior height point (H-ant) is proximally raised compared with the posterior height point (H-post) in respect of at least one sagittal plane; and
- an antero-medial height contour portion of the anterior height contour portion on the medial bearing shell (21) forms an anterior height point (H-ant), raised mostly proximally, as an antero-medial peak point (P).

2. The knee endoprosthesis (100) according to claim 1, wherein:
- the anterior height point (H-ant) forms an anterior bearing height, with respect to a sagittal plane, with respect to a proximally deepest bearing low point (T) in the concave medial and/or lateral bearing shell (21, 22); and
- the posterior height point (H-post) with respect to a sagittal plane forms a posterior bearing height (h), with respect to a proximally lowest bearing low point (T) in the concave medial and/or lateral bearing shell (21, 22);
**characterized in that** the anterior bearing height is greater than the posterior bearing height (h), further preferably the maximum anterior bearing height is formed at the antero-medial peak point (P).

3. The knee endoprosthesis (100) according to the directly preceding claim, wherein:
- in a medial sagittal plane, a pitch triangle spanning from the anterior height point (H-ant) with the anterior bearing height to the proximal lowest bearing low point (T) in the medial bearing shell (21) forms a medial pitch angle (β-med) located at the proximal lowest bearing low point (T);
**characterized in that** the medial pitch angle (β-med) measures 12 to 32 degrees, more preferably 17 to 27 degrees, in particular about 22 degrees.

4. The knee endoprosthesis (100) according to one of the preceding claims, wherein:
- a most distal point of the medial femur-condyle bearing surface (51) forms a medial extension bearing surface contact point which is in contact with the medial bearing shell (21) of the tibia bearing surface (15) at approximately 0 degrees of flexion angle;
**characterized in that** the medial extension bearing surface contact point,
- when at about 0 degrees flexion angle with respect to a sagittal plane as an angular segment related to an anterior edge of the medial femur-condyle bearing surface (51) is located at 50 to 70 degrees, more preferably at 58 to 62 degrees, in particular at about 60 degrees; and/or
- when at about 0 degrees flexion angle with respect to a sagittal plane as an angular segment related to an anterior edge of the medial bearing shell (21) of the tibial bearing surface (15) is located at 55 to 75 degrees, more preferably at 63 to 67 degrees, in particular at about 65 degrees.

5. The knee endoprosthesis (100) according to one of the preceding claims,
**characterized in that**
- the concave medial bearing shell (21) has, with respect to a medial sagittal plane, at least one anterior surface portion having a first radius forming an antero-medial tibia bearing surface radius (R1-med/ant), and at least one posterior surface portion having a second radius different from the first radius, preferably smaller, forming a postero-medial tibia bearing surface radius (R2-med/post); wherein in particular:
- the antero-medial tibia bearing surface radius (R1-med/ant) is congruent, in particular identical, to the anterior femur-condyle radius (R3), which is designated as a third radius of the anterior surface portion of the medial femur-condyle bearing surface (51), preferably predetermined according to a femur size class for a patient; and/or
- the postero-medial tibia bearing surface radius (R2-med/post) is congruent, in particular identical, to a posterior femur-condyle radius (R4), which is formed as a fourth radius of a posterior surface portion of the medial femur-condyle bearing surface (51), preferably predetermined according to a femur size class for a patient.

6. The knee endoprosthesis (100) according to one of the preceding claims,
**characterized in that**
- the concave lateral bearing shell (22) forms, with respect to a lateral sagittal plane, at least one anterior surface portion having a fifth radius forming an antero-lateral tibia bearing surface radius (R5-lat/ant) and at least one posterior surface portion having a sixth radius different from the fifth radius, preferably larger, forming a postero-lateral tibia bearing surface radius (R6-lat/post); wherein in particular, with reference to the immediately preceding claim:
- the antero-lateral tibia bearing surface radius (R5-lat/ant) is congruent, in particular identical, to the anterior femur-condyle radius (R3); and/or
- the postero-lateral tibia bearing surface radius (R6-lat/post) is larger than the posterior femur-condyle radius (R4), and is preferably at least 110 percent larger, in particular greater than or equal to 80 mm.

7. The knee endoprosthesis (100) according to one of claims 1 to 6, wherein:
- a postero-medial phase (60) is provided on the medial bearing shell (21) extending obliquely distally from the posterior height point (H-post) for peripheral material removal which, when projected in a sagittal plane, is at a posterior phase angle (δ) to a transverse plane;
**characterized in that** the postero-medial phase (60) is beveled with a posterior phase angle (δ) of 30 to 40 degrees, more preferably 33 to 37 degrees, in particular about 35 degrees.

8. The knee endoprosthesis (100) according to one of claims 1 to 7, wherein:
- a postero-lateral phase (70) for peripheral material removal is provided on the lateral bearing shell (22) and slopes distally at the posterior height point (H-post), said phase, when projected in a sagittal plane, is at a posterior phase angle (δ) to a transverse plane;
**characterized in that** the postero-lateral phase (70) is formed with a posterior phase angle (δ) of 5 to 15 degrees, more preferably of 8 to 12 degrees, in particular of about 10 degrees, and/or is rounded with a postero-lateral rounding radius (r) of 10 to 14 mm, more preferably of 11.2 to 12.8 mm, in particular of about 12 mm.

9. The knee endoprosthesis (100) according to one of the preceding claims, **characterized in that**
- the tibia bearing surface (15) forms an anterior patella bulge (25), which is provided centrally between the medial bearing shell (21) and the lateral bearing shell (22) and which has a concave outer body contour; and in particular the patella bulge (25) is formed asymmetrically and/or congruent to a patella size class predetermined for a patient and/or a statistically-anthropometrically predetermined 50 percentile value for a patella size distribution with respect to a representative population.

10. The knee endoprosthesis (100) according to one of the preceding claims, **characterized in that** the medial femur-condyle bearing surface (51) and the lateral femur-condyle bearing surface (52) form a femur bearing surface (55) which is substantially mirror-symmetrical with respect to a sagittal plane arranged therebetween.

11. The knee endoprosthesis (100) according to one of the preceding claims, **characterized in that** the medial femur-condyle bearing surface (51) has, at least in a distal-medial surface section, a medial femur-condyle bearing surface radius, in particular for imitating a natural medial femoral condyle radius, which remains constant; wherein the lateral femur-condyle bearing surface (52) has, at least in a lateral-medial surface portion, another lateral femur-condyle bearing surface radius, in particular for simulating a natural lateral femoral condyle radius, remaining constant; and wherein the medial femur-condyle bearing surface radius and the lateral femur-condyle bearing surface radius differ slightly from each other at most by a ratio factor of from 0.8 to 1.2, preferably from 0.9 to 1.1, in particular from 0.95 to 1.05.

12. The knee endoprosthesis according to one of the preceding claims, **characterized in that** the tibia bearing surface (15) is arranged for receiving and displaceably slidingly supporting the femur bearing surface (55) along a guiding curvature line (a, b, c) formed by a plurality of contact points, so that upon flexing the knee endoprosthesis (100), preferably under load of a standard body weight of 75kg, from 0 degrees to about 90 degrees flexion angle:
- an amplitude of minus 0 mm to minus 11 mm femoral roll-back is effected, wherein in a preferred manner the femoral roll-back, when referred to a forward positive coordinate axis, is less than or equal to minus 1.5 mm at 30 degrees and/or less than or equal to minus 6 mm at 60 degrees and/or less than or equal to minus 11 mm at about 90 degrees flexion angle.

13. The knee endoprosthesis according to one of the preceding claims, **characterized in that** the tibia bearing surface (15) is arranged for receiving and displaceably slidingly supporting the femur bearing surface (55), so that during a flexing bending of the knee endoprosthesis (100) under a gait cycle, in particular a movement sequence of 0-15-0-60 degrees flexion angle and preferably during a load in gait with a standard body weight of 75kg:
- causing a medial movement of the medial femoral condyle by delta 1 mm to 4 mm, preferably substantially 2 mm; and
- a lateral movement of the lateral femoral condyle is caused by delta 6 mm to 11 mm, preferably essentially 9 mm, so that
in a preferred manner during flexion of the knee endoprosthesis (100) under a gait cycle of 0-15-0-60 degrees flexion angle and preferably during loading in gait with a norm body weight of 75kg:
- causes a movement of the medial femoral condyle, from 0 mm to a maximum of 2 mm anteriorly and back again from 2 mm to 0 mm posteriorly; and
- a movement of the lateral femoral condyle by 5.5 mm anteriorly, by 9 mm posteriorly and again by 3.5 mm anteriorly is caused.

14. An inlay (20) which is arranged proximally on the tibia part (10) of a knee endoprosthesis (100) according to one of the preceding claims, in particular formed integrally with the tibia part (10), and/or can be arranged,
- wherein a proximal surface of the inlay (20) forms the tibia bearing surface (15) having a concave medial bearing shell (21) and a concave lateral bearing shell (22), which is adapted to receive and slidably support the femur bearing surface (55) having a convex medial femur condyle bearing surface (51) and a convex lateral femur condyle bearing surface (52) without a fixed medial pivot point of the femur bearing surface (55) in the medial bearing shell (21);
- wherein the medial bearing shell (21) and the lateral bearing shell (22) form an asymmetrical tibia bearing surface (15) relative to each other;
- the medial femur-condyle bearing surface (51) and the lateral femur-condyle bearing surface (52) form a non-asymmetrical, in particular a mirror-symmetrical, femur bearing surface (55) with respect to a sagittal plane arranged therebetween
**characterized in that**
- the medial femur-condyle bearing surface (51) and the lateral femur-condyle bearing surface (52) coincide at least in an anterior femur-condyle radius (R3) of respective anterior surface portions;
- the asymmetric tibia bearing surface (15) forms an outer circumferential proximal height contour;
- the height contour comprises an anterior height contour portion having at least one anterior height point (H-ant) and a posterior height contour portion having at least one posterior height point (H-post), wherein the anterior height point (H-ant) is proximally elevated with respect to the posterior height point (H-post) with respect to at least one sagittal plane; and
- an antero-medial height contour portion of the anterior height contour portion at the medial bearing shell (21) forms a proximally most elevated anterior height point (H-ant) as an antero-medial peak point (P).

## Revendications

1. Endoprothèse de genou (100) à stabilisation médiale pour une arthroplastie totale du genou avec conservation ou avec dissection du ligament croisé postérieur comportant :
- une surface d'appui de fémur (55) qui est prévue au niveau d'une partie pour fémur (50) conçue pour être fixée au niveau d'une extrémité distale d'un fémur et pour une orientation vers le côté distal ; et
- une surface d'appui de tibia (15) qui est prévue au niveau d'une partie pour tibia (10) conçue pour être fixée au niveau d'une extrémité proximale d'un tibia et pour une orientation vers le côté proximal, dans laquelle la surface d'appui de tibia (15) est en particulier formée comme une surface proximale d'un inlay (20) disposé et/ou pouvant être disposé de manière proximale au niveau de la partie pour tibia (10),
- dans laquelle la surface d'appui de fémur (55) présente une surface d'appui de condyle fémoral médiale (51) convexe et une surface d'appui de condyle fémoral latérale (52) convexe ;
- dans laquelle la surface d'appui de tibia (15) présente une coque d'appui médiale (21) concave et une coque d'appui latérale (22) concave et est conçue pour la réception et la formation d'un palier lisse coulissant de la surface d'appui de fémur (55) sans point de rotation médial fixe de la surface d'appui de fémur (55) dans la coque d'appui médiale (21) ;
- dans laquelle la coque d'appui médiale (21) et la coque d'appui latérale (22) forment, l'une par rapport à l'autre, une surface d'appui de tibia (15) asymétrique ; et
- la surface d'appui de condyle fémoral médiale (51) et la surface d'appui de condyle fémoral latérale (52) forment, l'une par rapport à l'autre, une surface d'appui de fémur (55) non asymétrique ou quasi-symétrique,
**caractérisée en ce que**
- la surface d'appui de condyle fémoral médiale (51) et la surface d'appui de condyle fémoral latérale (52) correspondent, au moins dans un rayon de condyle fémoral antérieur (R3), à des sections de surface antérieures respectives ;
- la surface d'appui de tibia (15) asymétrique forme un contour de hauteur proximal circonférentiel extérieur ;
- le contour de hauteur présente une section de contour de hauteur antérieure comportant au moins un point de hauteur antérieur (H-ant) et une section de contour de hauteur postérieure comportant au moins un point de hauteur postérieur (H-post), dans laquelle le point de hauteur antérieur (H-ant) est plus élevé côté proximal par rapport au point de hauteur postérieur (H-post) en ce qui concerne au moins un plan sagittal ; et
- une section de contour de hauteur antéro-médiale de la section de contour de hauteur antérieure forme, au niveau de la coque d'appui médiale (21), un point de hauteur antérieur (H-ant) le plus élevé côté proximal en tant que point culminant antéro-médial (P).

2. Endoprothèse de genou (100) selon la revendication 1, dans laquelle :
- le point de hauteur antérieur (H-ant), en ce qui concerne un plan sagittal, forme une hauteur d'appui antérieure par rapport à un point bas d'appui le plus bas côté proximal (T) dans la coque d'appui médiale et/ou latérale (21, 22) concave ; et
- le point de hauteur postérieur (H-post), en ce qui concerne un plan sagittal, forme une hauteur d'appui postérieure (h) par rapport à un point bas d'appui le plus bas côté proximal (T) dans la coque d'appui médiale et/ou latérale (21, 22) concave ;
**caractérisée en ce que** la hauteur d'appui antérieure est supérieure à la hauteur d'appui postérieure (h), plus préférablement la hauteur d'appui antérieure maximale est formée au niveau du point culminant antéro-médial (P).

3. Endoprothèse de genou (100) selon la revendication immédiatement précédente, dans laquelle :
- dans un plan sagittal médial, un triangle d'inclinaison, lequel s'étend du point de hauteur antérieur (H-ant) comportant la hauteur d'appui antérieure au point bas d'appui le plus bas côté proximal (T) dans la coque d'appui médiale (21), forme un angle d'inclinaison médial (β-med) disposé au niveau du point bas d'appui le plus bas côté proximal (T) ;
**caractérisée en ce que** l'angle d'inclinaison médial (β-med) mesure 12 à 32 degrés, plus préférablement 17 à 27 degrés, en particulier environ 22 degrés.

4. Endoprothèse de genou (100) selon l'une des revendications précédentes, dans laquelle :
- un point le plus distal de la surface d'appui de condyle fémoral médiale (51) forme un point de contact de surface d'appui d'extension médial en contact avec la coque d'appui médiale (21) de la surface d'appui de tibia (15) à un angle de flexion d'environ 0 degré ;
**caractérisée en ce que** le point de contact de surface d'appui d'extension médial,
- lorsqu'il est considéré, à un angle de flexion d'environ 0 degré en ce qui concerne un plan sagittal, en tant que segment d'angle par rapport à un bord antérieur de la surface d'appui de condyle fémoral médiale (51), est disposé à 50 jusqu'à 70 degrés, plus préférablement à 58 jusqu'à 62 degrés, en particulier à environ 60 degrés ; et/ou
- lorsqu'il est considéré, à un angle de flexion d'environ 0 degré en ce qui concerne un plan sagittal, en tant que segment d'angle par rapport à un bord antérieur de la coque d'appui médiale (21) de la surface d'appui de tibia (15), est disposé à 55 jusqu'à 75 degrés, plus préférablement à 63 jusqu'à 67 degrés, en particulier à environ 65 degrés.

5. Endoprothèse de genou (100) selon l'une des revendications précédentes, **caractérisée en ce que**
- la coque d'appui médiale (21) concave, en ce qui concerne un plan sagittal médial, forme au moins une section de surface antérieure comportant un premier rayon, lequel forme un rayon de surface d'appui de tibia antéro-médial (R1-med/ant), et au moins une section de surface postérieure comportant un deuxième rayon différent du premier rayon, de préférence plus petit que celui-ci, lequel forme un rayon de surface d'appui de tibia postéro-médial (R2-med/post) ; dans laquelle, en particulier :
- le rayon de surface d'appui de tibia antéro-médial (R1-med/ant) est formé de manière congruente, en particulier identique, au rayon de condyle fémoral antérieur (R3) qui est désigné comme un troisième rayon de la section de surface antérieure de la surface d'appui de condyle fémoral médiale (51), de préférence est prédéfini selon une classe de dimensions de fémur pour un patient ; et/ou
- le rayon de surface d'appui de tibia postéro-médial (R2-med/post) est formé de manière congruente, en particulier identique, à un rayon de condyle fémoral postérieur (R4) qui est réalisé comme un quatrième rayon d'une section de surface postérieure de la surface d'appui de condyle fémoral médiale (51), de préférence est prédéfini selon une classe de dimensions de fémur pour un patient.

6. Endoprothèse de genou (100) selon l'une des revendications précédentes, **caractérisée en ce que**
- la coque d'appui latérale (22) concave, en ce qui concerne un plan sagittal latéral, forme au moins une section de surface antérieure comportant un cinquième rayon, lequel forme un rayon de surface d'appui de tibia antéro-latéral (R5-lat/ant), et au moins une section de surface postérieure comportant un sixième rayon différent du cinquième rayon, de préférence supérieur à celui-ci, lequel forme un rayon de surface d'appui de tibia postéro-latéral (R6-lat/post) ; dans laquelle, en particulier, en référence à la revendication immédiatement précédente :
- le rayon de surface d'appui de tibia antéro-latéral (R5-lat/ant) est formé de manière congruente, en particulier identique, au rayon de condyle fémoral antérieur (R3) ; et/ou
- le rayon de surface d'appui de tibia postéro-latéral (R6-lat/post) est formé de manière à être plus grand que le rayon de condyle fémoral postérieur (R4), plus préférablement est dimensionné de manière à être supérieur d'au moins 110 pour cent et est en particulier supérieur ou égal à 80 mm.

7. Endoprothèse de genou (100) selon l'une des revendications 1 à 6, dans laquelle :
- au niveau de la coque d'appui médiale (21), une phase postéro-médiale (60) s'étendant en biais vers le côté distal à partir du point de hauteur postérieur (H-post) est prévue pour le retrait de matériau périphérique, laquelle phase, lorsqu'elle est projetée dans un plan sagittal, se trouve à un angle de phase postérieur (δ) par rapport à un plan transversal ;
**caractérisée en ce que** la phase postéro-médiale (60) est inclinée d'un angle de phase postérieur (δ) de 30 à 40 degrés, plus préférablement de 33 à 37 degrés, en particulier d'environ 35 degrés.

8. Endoprothèse de genou (100) selon l'une des revendications 1 à 7, dans laquelle :
- au niveau de la coque d'appui latérale (22), une phase postéro-latérale (70) s'étendant en biais vers le côté distal au niveau du point de hauteur postérieur (H-post) est prévue pour le retrait de matériau périphérique, laquelle phase, lorsqu'elle est projetée dans un plan sagittal, se trouve à un angle de phase postérieur (δ) par rapport à un plan transversal ;
**caractérisée en ce que** la phase postéro-latérale (70) est formée à un angle de phase postérieur (δ) de 5 à 15 degrés, plus préférablement de 8 à 12 degrés, en particulier d'environ 10 degrés, et/ou est arrondie à un rayon de courbure postéro-latéral (r) de 10 à 14 mm, plus préférablement de 11,2 à 12,8 mm, en particulier d'environ 12 mm.

9. Endoprothèse de genou (100) selon l'une des revendications précédentes, **caractérisée en ce que**
- la surface d'appui de tibia (15) forme une protubérance pour rotule antérieure (25), prévue centralement entre la coque d'appui médiale (21) et la coque d'appui latérale (22) et comportant un contour de corps externe concave ; et, en particulier, la protubérance pour rotule (25) est formée de manière asymétrique et/ou est formée de manière congruente par rapport à une classe de dimensions de rotule prédéfinie pour un patient et/ou à une valeur de percentile 50 prédéfinie de manière statistique-anthropométrique pour une distribution de dimensions de rotule en ce qui concerne une population représentative.

10. Endoprothèse de genou (100) selon l'une des revendications précédentes, **caractérisée en ce que** la surface d'appui de condyle fémoral médiale (51) et la surface d'appui de condyle fémoral latérale (52) forment, l'une par rapport à l'autre, une surface d'appui de fémur (55) sensiblement symétrique en miroir en ce qui concerne un plan sagittal disposé entre elles.

11. Endoprothèse de genou (100) selon l'une des revendications précédentes, **caractérisée en ce que** la surface d'appui de condyle fémoral médiale (51) présente constamment, au moins dans une section de surface distale-médiale, un rayon de surface d'appui de condyle fémoral médial, en particulier pour la recréation d'un rayon de condyle fémoral médial naturel ; dans laquelle la surface d'appui de condyle fémoral latérale (52) présente constamment, au moins dans une section de surface latérale-médiale, un autre rayon de surface d'appui de condyle fémoral latéral, en particulier pour la recréation d'un rayon de condyle fémoral latéral naturel ; et dans laquelle le rayon de surface d'appui de condyle fémoral médial et le rayon de surface d'appui de condyle fémoral latéral s'écartent légèrement l'un de l'autre au maximum d'un facteur de rapport de 0,8 à 1,2, de préférence de 0,9 à 1,1, en particulier de 0,95 à 1,05.

12. Endoprothèse de genou selon l'une des revendications précédentes, **caractérisée en ce que** la surface d'appui de tibia (15) est conçue pour la réception et la formation d'un palier lisse coulissant de la surface d'appui de fémur (55) le long d'une ligne de courbure conductrice (a, b, c) formée par une pluralité de points de contact, de telle sorte que, en cas de fléchissement de l'endoprothèse de genou (100), de préférence sous la charge d'un poids corporel normalisé de 75 kg, à un angle de flexion de 0 degré à environ 90 degrés :
- une amplitude de moins 0 mm à moins 11 mm de recul fémoral est provoquée, dans laquelle, de manière préférée, le recul fémoral, lorsqu'il est considéré par rapport à un axe de coordonnées positif dans le sens avant, est inférieur ou égal à moins 1,5 mm à un angle de flexion de 30 degrés et/ou inférieur ou égal à moins 6 mm à un angle de flexion de 60 degrés et/ou inférieur ou égal à moins 11 mm à un angle de flexion d'environ 90 degrés.

13. Endoprothèse de genou selon l'une des revendications précédentes, **caractérisée en ce que** la surface d'appui de tibia (15) est conçue pour la réception et la formation d'un palier lisse coulissant de la surface d'appui de fémur (55) de telle sorte que, en cas de fléchissement de l'endoprothèse de genou (100) lors d'un cycle de marche, en particulier d'une séquence de mouvements à un angle de flexion de 0-15-0-60 degré et de préférence en cas de charge lors de la marche par un poids corporel normalisé de 75 kg :
- un mouvement médial du condyle fémoral médial de delta 1 mm à 4 mm, de préférence de sensiblement 2 mm, est provoqué ; et
- un mouvement latéral du condyle fémoral latéral de delta 6 mm à 11 mm, de préférence de sensiblement 9 mm, est provoqué, de sorte que, de manière préférée, en cas de fléchissement de l'endoprothèse de genou (100) lors d'un cycle de marche à un angle de flexion de 0-15-0-60 degré et de préférence en cas de charge lors de la mache par un poids corporel normalisé de 75 kg :
- un mouvement du condyle fémoral médial déplacé de 0 mm à maximum 2 mm en mouvement antérieur et de nouveau au retour de 2 mm à 0 mm en mouvement postérieur est provoqué ; et
- un mouvement du condyle fémoral latéral de 5,5 mm en mouvement antérieur, de 9 mm en mouvement postérieur et de nouveau de 3,5 mm en mouvement antérieur est provoqué.

14. Inlay (20), lequel est disposé de manière proximale au niveau de la partie pour tibia (10) d'une endoprothèse de genou (100) selon l'une des revendications précédentes, en particulier est formé de manière solidaire avec la partie pour tibia (10), et/ou est conçu de manière à pouvoir être disposé,
- dans lequel une surface proximale de l'inlay (20) forme la surface d'appui de tibia (15) présentant une coque d'appui médiale (21) concave et une coque d'appui latérale (22) concave, laquelle surface d'appui de tibia est conçue pour la réception et la formation d'un palier lisse coulissant de la surface d'appui de fémur (55) présentant une surface d'appui de condyle fémoral médiale (51) convexe et une surface d'appui de condyle fémoral latérale (52) convexe, sans point de rotation médial fixe de la surface d'appui de fémur (55) dans la coque d'appui médiale (21) ;
- dans lequel la coque d'appui médiale (21) et la coque d'appui latérale (22) forment, l'une par rapport à l'autre, une surface d'appui de tibia (15) asymétrique ;
- la surface d'appui de condyle fémoral médiale (51) et la surface d'appui de condyle fémoral latérale (52) forment, l'une par rapport à l'autre, une surface d'appui de fémur (55) non asymétrique, en particulier symétrique en miroir en ce qui concerne un plan sagittal disposé entre elles,
**caractérisé en ce que**
- la surface d'appui de condyle fémoral médiale (51) et la surface d'appui de condyle fémoral latérale (52) correspondent, au moins dans un rayon de condyle fémoral antérieur (R3), à des sections de surface antérieures respectives ;
- la surface d'appui de tibia (15) asymétrique forme un contour de hauteur proximal circonférentiel extérieur ;
- le contour de hauteur présente une section de contour de hauteur antérieure comportant au moins un point de hauteur antérieur (H-ant) et une section de contour de hauteur postérieure comportant au moins un point de hauteur postérieur (H-post), dans lequel le point de hauteur antérieur (H-ant) est plus élevé côté proximal par rapport au point de hauteur postérieur (H-post) en ce qui concerne au moins un plan sagittal ; et
- une section de contour de hauteur antéro-médiale de la section de contour de hauteur antérieure forme, au niveau de la coque d'appui médiale (21), un point de hauteur antérieur (H-ant) le plus élevé côté proximal en tant que point culminant antéro-médial (P).
